# EUROPEAN PATENT SPECIFICATION

(11) **EP 0 337 942 B1**
(45) Date of publication and mention of the grant of the patent: **06.07.1994**
(21) Application number: 89810250.4
(22) Date of filing: 04.04.1989
(51) Int. Cl.: C07D 249/20, C08K 5/34, C09K 15/30

(54) **Liquid substituted 2H-benzotriazole mixtures**
Flüssige Mischungen substituierter 2H-Benzotriazole
Mélanges liquides de 2H-benzotriazoles substitués

(30) Priority: 11.04.1988 US 179737
(43) Date of publication of application: 18.10.1989
(73) Proprietor: CIBA-GEIGY AG, 4002 Basel (CH)
(72) Inventor: Winter, Roland A. E., Armonk New York 10504 (US); Detlefsen, Robert E., Putnam Valley New York 10579 (US); Stegmann, Werner, Dr., CH-4410 Liestal (CH); Luisoli, Reto, CH-4434 Hölstein (CH); Leppard, David, Dr., CH-1723 Marly (CH)

(56) References cited:
- EP-A- 0 189 374
- DE-B- 2 130 322
- US-A- 3 983 132
- US-A- 4 278 590

## Description

The present invention relates to selected liquid 2-hydroxy-phenyl-2H-benzotriazole mixtures which are useful in protecting light-sensitive organic materials from deterioration and to stabilized compositions containing said benzotriazoles, to a process of preparing them and to stabilized materials containing them.

The UV-absorbers of the 2-hydroxyphenyl-2H-benzotriazole class have long been known as effective light stabilizers for organic materials and have enjoyed considerable commercial success.

The description, preparation and uses of such valuable 2-aryl-2H-benzotriazolesare further taught in U.S. Patent Nos. 3,004,896; 3,055,896; 3,072,585; 3,074,910; 3,189,615; 3,230,194; and in particular EP-A-0 189374.

However, the hitherto known 2-aryl-2H-benzotriazoles of this group have in some circumstances exhibited limited compatibility in certain substrates, and excessive tendency to exude, sublime and/or volatilize during processing of stabilized compositions into sheets, films, fibers or other pellicles when processing must be done at elevated temperatures. Likewise such benzotriazoles may also suffer undue loss by volatilization or sublimation from fabricated structures, particularly thin films or coatings, especially when subjected to elevated temperatures during use.

Attempts have been made to increase compatibility and to reduce volatilization loss by modifying the structure of the benzotriazoles.

In U.S. Patent No. 3,230,194, a higher alkyl group was substituted for methyl and the latter compound 2-(2-hydroxy-5-tert-octylphenyl)-2H-benzotriazole exhibited superior compatibility and performance in polyethylene compared to the former.

In U.S. Patent Nos. 4,283,327, 4,278,590 and 4,383,863 there is described 2-(2-hydroxy-3,5-di-tert-octylphenyl)-2H-benzotriazole which exhibits an excellent combination of compatibility with and/or solubility in numerous polymeric substrates along with superior resistance to loss from stabilized compositions during high temperature processing or in end-use applications where coating or films of the stabilized compositions are exposed even to ambient weathering and light exposures, and in photographic applications. However, 2-(2-hydroxy-3,5-di-tert-octylphenyl)-2H-benzotriazole is still a solid (melting point 105 - 106 _{°} C) which requires in many end-use applications the concomitant use of a solvent or dispersing diluent to allow for it to be used in practice. Such solvents or diluents are undesired for reasons of cost and environmental and other considerations.

U.S. Patent Nos. 3,983,132, 4,096,242 and 4,129,521 describe liquid mixtures of 2-(2-hydroxy-5-nonylphenyl)-2H-benzotriazoles or of 2-(2-hydroxy-5-dodecylphenyl)-2H-benzotriazoles and stabilized compositions using said mixtures where the nonyl or dodecyl groups each represent an isomeric mixture of secondary and tertiary nonyl or dodecyl groups attached to the para position relevant to the hydroxy group on the 2-phenyl moiety in the 2H-benzotriazole. The isomeric nonyl or dodecyl groups are introduced into the phenol before it is coupled with the 2-nitrophenyldiazonium salt in a classic 2H-benzotriazole synthesis.

The instant liquid benzotriazoles differ from the benzotriazoles of these three patents by the method by which they are prepared, by the location of the branched alkyl group ortho to the hydroxy group and by in part the nature of the branched alkyl group itself when prepared from a straight chain alkene.

The liquid mixtures prepared by the method of U.S. Patent No. 4,129,521 have no substitution in the ortho position relevant to the hydroxy group thus making said compounds prone to interaction with metal ions during resin curing and in other end-use applications in polymer substrates and which may lead to deleterious effects on color, light stability and ancillary properties. The instant mixtures are substituted in the ortho position relevant to the hydroxyl group and do not have this problem.

Certain hydrophobic non-diffusing hydroxyphenylbenzotriazoles are disclosed as very useful as ultraviolet light absorbers in photographic gelatin layers (U.S. Patent No. 3,253,921). The instant benzotriazoles with their liquid or non-crystalline nature, their desirable absorption characteristics in the ultraviolet range and their photographic inertness are particularly useful in photographic compositions, especially in protecting color dye images against the harmful effects of ultraviolet light.

U.S. Patent No. 3,253,921 discloses benzotriazoles broadly, but does not exemplify the instant benzotriazoles which are particularly effective in stabilizing photographic compositions against the harmful effects of ultra-violet radiation.

Further background in the area of stabilization of photographic dye images is provided by U.S. Patent No. 4,042,394 which describes the various components in photographic compositions and the requirements for stabilizing photographic dye images.

U.S. Patent Nos. 4,383,863 and 4,447,511 describe the use of 2-(2-hydroxy-3,5-di-tert-octylphenyl)-2H-benzotriazole in photographic elements and compositions. While this discrete benzotriazole exhibits enhanced solubility in the various solvents and diluents used in photographic elements, such solvents and diluents are still required since said benzotriazole is still a crystalline solid.

The instant benzotriazole mixtures are liquid or non-crystalline leading to the need for less or no solvent or diluent, thinner photographic layers and all the concomitant economic benefits flowing therefrom.

U.S. Patent No. 4,127,586 teaches that it is possible to alkylate phenols with long chain olefins to get a phenol substituted with a mixture of isomers as the alkyl substituents. The corresponding 2H-benzotriazole is then prepared by the classic diazotizing, coupling and reduction route starting with o-nitroaniline. The 2H-benzotriazole prepared by this classic method differs in the distribution of isomeric components and in chemical properties from the instant products made by an entirely different process.

U.S. Patent Nos. 4,587,346 and 4,675,352 pertain to the alkylation of preformed 2-(2-hydroxy-5-methylphenyl)-2H-benzotriazole using a long straight or branched alkene in the presence of an acid catalyst at 100 - 200 °C. Liquid mixtures are obtained by this process, but the final products always contain a 5-methyl substituent.

This invention pertains to selected liquid or non-crystalline 2-aryl-2H-benzotriazole light absorbers and to organic materials, both polymeric and non-polymeric, stabilized thereby, as well as to photographic elements containing said liquid materials. The stabilized compositions include plastics, coatings, fibers, films, and photographic substrates.

Another object of this invention is the process for preparing said liquid or non-crystalline mixtures of benzotriazoles. These liquid mixtures exhibit great resistance to volatilization, enhanced solubility in selected solvents, desirable absorption characteristics in the ultraviolet range and photographic inertness. This combination of properties makes these benzotriazoles particularly useful in photographic compositions especially in protecting color dye images against the harmful effects of ultraviolet light.

In a first aspect the present invention consists in a process for preparing a normally liquid or non-crystalline mixture of 2-hydroxyphenyl-2H-benzotriazoles which comprises reacting a 2H-benzotriazole of the formula (A) or (B) 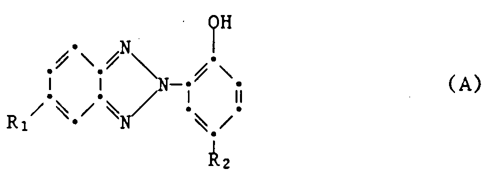 or 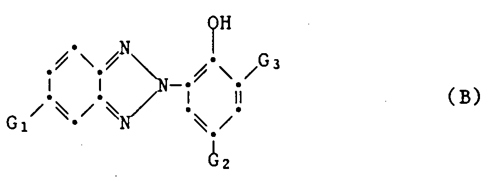 wherein
R₁ is hydrogen, chloro, alkyl of 1 to 4 carbon atoms or alkoxy of 1 to 4 carbon atoms,
R₂ is alkyl of 2 to 12 carbon atoms, cycloalkyl of 5 to 8 carbon atoms, aralkyl of 7 to 9 carbon atoms or chlorine,
G₁ has the same meaning as Ri, and
G₂ and G₃ are independently branched alkyl of 3 to 12 carbon atoms, cycloalkyl of 5 to 8 carbon atoms or aralkyl of 7 to 9 carbon atoms, or one of G₂ and G₃ is methyl, ethyl or chlorine when the other of G₂ and G₃ is branched alkyl, cycloalkyl or aralkyl as defined above, with a straight or branched chain alkene of 8 to 40 carbon atoms or mixture of said alkenes in the presence of an acidic catalyst at a temperature of 100 to 200 _{°} C.

Preferably, R₁ and G₁ are hydrogen or chloro; most preferably hydrogen.

Preferably R₂ or G₂ and G₃ are independently branched alkyl of 3 to 12 carbon atoms or aralkyl of 7 to 9 carbon atoms, or R₂ is ethyl or chlorine, or one of G₂ or G₃ is methyl, ethyl or chlorine and the other of G₂ or G₃ is branched alkyl of 3 to 12 carbon atoms.

When R₂, G₂ or G₃ is branched alkyl of 3 to 12 carbon atoms, said alkyl is for example isopropyl, sec- butyl, tert-butyl, tert-amyl, tert-octyl or tert-dodecyl. When R₂, G₂ or G₃ is aralkyl of 7 to 9 carbon atoms, said aralkyl is e.g. benzyl, alpha-methylbenzyl or alpha,alpha-dimethylbenzyl.

Further preferred starting materials of formula (A) or (B) are those wherein R₂ is branched C₄ to C₈ alkyl or wherein at least one of G₂ or G₃ is branched C₄ to C₈-alkyl, preferably wherein both G₂ and G₃ are branched, especially tertiary alkyl, as well as those of formula (A) wherein R₂ is ethyl or chlorine.

Under these vigorous reaction conditions the alkylating agent (the alkene, straight or branched chain) itself undergoes a chemical transformation or isomerization. Accordingly the alkyl substituents introduced into the benzotriazole are not a single discrete moiety, but rather a random statistical mixture of isomeric groups. This random statistical mixture of groups represents a structural diversity which contributes to the liquid and non-crystalline physical state of the resulting products.

Under the instant process conditions the double bond in the alkene alkylating agent is isomerized along the carbon chain to give a random statistical mixture of moieties which can then be attached to the phenolic ring in the benzotriazole.

Illustrating with the alpha-olefin 1-octene, the random statistical mixture of octyl groups which are introduced into the molecule are, for example, 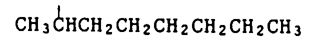 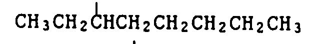 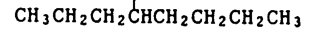 etc.

Indeed, under the instant process conditions, the alkene being used in the instant process may fragment, dimerize or recombine in some fashion before reacting with the 2H-benzotriazole starting material. Such possibilities may in part account for the wide mixture of molecular species noted in the working Examples 1 - 11 where mass spectrographic analysis shows the presence of inter alia species with alkyl substituents having twice the number of carbon atoms as were present in the original alkene being used. In like manner, mass spectrographic analysis indicated some species with alkyl substituents having less than the number of carbon atoms in the alkene being used in the instant process or even having less than the number of carbon atoms in the alkyl groups in the original 2H-benzotriazole starting material used. In some cases, there were species having no alkyl substituents at all with complete dealkylation of the original 2H-benzotriazole having occurred. Inspection of the mass spectrographic analysis data shows that the combined amounts of all of these possible species enumerated in this paragraph are essentially minor components in the instant mixtures.

Although clearly the alkyl groups introduced into the molecule are in an overwhelming degree present at the 3- and 5-positions (ortho and para positions) of the phenolic ring, it cannot be ruled out that some substitution at the meta (4-) position or even in the benzo ring may not also occur in minor and inconsequential amounts.

In the case where the starting 2H-benzotriazole has a lower alkyl substituent on the benzo ring as R₁ or G, , said alkyl substituent is also unlikely to participate in the instant process since said benzo ring is quite deactivated and is not prone to direct alkylation and to participation in the instant process.

The alkenes useful in the instant process are any alkene whether straight or branched chain including alpha-olefins and alkenes having an internal double bond. During the instant alkylation process the double bond is isomerized along the carbon chain to give a random statistical mixture of alkenes which in turn produce a random statistical mixture of alkyl substituents.

Further contributing to the random statistical nature of the alkyl substituents is the fact that commercially available alkenes are often mixture of alkenes.

Preferred alkenes useful in the instant process are those of 8 to 30, especially 8 to 20, preferably 8 to 16, in particular 8 to 14, for example 8 to 12 carbon atoms. The most preferred alkenes are a-olefins.

The alpha-olefins useful in this process are for example 1-octene, 1-nonene, 1-decene, 1-undecene, 1- dodecene, 1-tridecene, 1-tetradecene, 1-hexadecene, 1-octadecene, 1-eicosene, 1-docosene, 1-tetracosene or 1-triacontene.

These alpha-olefins are largely items of commerce or are made by the telomerization of ethylene by known methods.

Straight chain alkenes containing an internal double bond may be for example 2-octene, 4-octene, 5- decene or 9-tricosene. These alkenes are also largely items of commerce.

The branched chain alkenes useful in this process are for example dipropylene, tripropylene, tetrapropylene, pentapropylene, diisobutylene, triisobutylene, tetraisobutylene, pentaisobutylene, 2,2,4,6,6-pentamethyl-3-heptene, diisoamylene, triisoamylene, tetraisoamylene or pentaisoamylene.

These highly branched alkenes are largely items of commerce or can be prepared from propylene, isobutylene or isoamylene by oligomerization with acid catalysts.

That this mixture of isomeric radicals is critical to obtaining a liquid or non-crystalline product may be seen from the fact that, when the alkyl substitution is a specific isomer, solid crystalline products are obtained. For example, 2-(2-hydroxy-3,5-di-tert-octylphenyl)-2H-benzotriazole melts at 105 - 106 _{°} C.

The alkylation processes can be carried out over a range of conditions of time, temperature, olefin to benzotriazole ratios, catalysts and catalyst concentrations.

The alkylation time can vary in a wide range, for example from about 15 minutes to 12 hours. Reaction times in excess of 12 hours do not increase yield of alkylated product. Preferably the alkylation reaction is carried out for a 30 minutes to 8-hour period.

Relatively vigorous reaction conditions are needed since even the phenolic ring of the starting 2H-benzotriazole is deactivated. Reaction temperatures of 100 to 200 °C are used. Preferably the process is carried out at 140 to 180°C, and most preferably at 160 - 175 °C.

In order to alkylate the 2H-benzotriazole there must be at least 1 equivalent of alkene per equivalent of 2H-benzotriazole. Since competing reactions are also possible under these reaction conditions in respect to the alkene, such as dimerization, oligomerization or polymerization, it is preferred to use an excess of alkene.

The equivalent ratio of alkene to 2H-benzotriazole of formula (A) or (B) is advantageously in the range of 1:1 to 6:1, preferably 2 to 4.5:1, for example 2.5 to 4.5:1.

The acidic catalyst is selected from the group consisting of aliphatic, aromatic and substituted aromatic sulfonic acids, sulfuric acid, phosphoric acid, acidic clays and heterogenous acidic catalysts (molecular sieves). Preferred are aliphatic sulfonic acids.

The concentration of catalyst useful in the instant process is, for example, 0.2 to 3 equivalents of catalyst per equivalent by benzotriazole, preferably 0.3 to 2 equivalents, and most preferably 0.5 to 1 equivalent of acid catalyst per equivalent of benzotriazole.

Examples of useful sulfonic acids are methanesulfonic acid, ethanesulfonic acid, butanesulfonic acid, benzenesulfonic acid, toluenesulfonic acid and dodecylbenzenesulfonic acid.

Commercially available acid activated clays such as Filtrol XJ-8303; Filtrol XJ-8405; Filtrol 22; Filtrol 4; and Filtrol 13 are also effective alkylation catalysts in the instant processes.

The most preferred catalyst is methanesulfonic acid.

The resulting mixtures of alkylated benzotriazoles may be isolated following the alkylation reaction by a number of methods including the extraction procedures described in U.S. Patent Nos. 4,587,346 and 4,675,352. Another facile method involves vacuum distillation at reduced pressure. An advantageous method is distillation in a short-path distillation apparatus.

A further aspect of the instant invention is the normally liquid or non-crystalline mixture of benzotriazoles obtainable according to the process according to this invention described hereinbefore.

The probable composition of the mixtures obtainable by the process of this invention is given by formula I 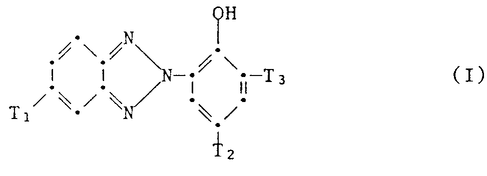 wherein
T₁ is hydrogen, chloro, alkyl of 1 to 4 carbon atoms or alkoxy of 1 to 4 carbon atoms, and in major proportions compounds
where one of T₂ or T₃ is a random statistical mixture of at least three isomeric alkyl groups each having 8 to 40 carbon atoms, and
the other of T₂ or T₃ is hydrogen or methyl, and
where one of T₂ or T₃ is a random statistical mixture as defined above and the other of T₂ or T₃ is alkyl of 2 to 12 carbon atoms, cycloalkyl of 5 to 8 carbon atoms, aralkyl of 7 to 9 carbon atoms or chlorine; and in minor proportions compounds wherei both of T₂ and T₃ are a random statistical mixture as defined above or where the compounds represent dealkylated or fragmented-alkyl substituted products of the benzotriazoles of formula I. The mixtures are obtainable by reacting a 2H-benzotriazole of formula (A) or (B) defined hereinbefore with a straight or branched chain alkene of 8 to 40 carbon atoms or mixture of said alkenes in the presence of an acidic catalyst at a temperature of 100 to 200 _{°} C.

Preferably T₁ is hydrogen or chloro; most preferably hydrogen.

Preferably T₂ or T₃ in the random statistical mixture of alkyl groups is alkyl of 8 to 30, especially 8 to 20, in particular 8 to 16 carbon atoms; most preferably alkyl of 8 to 14, for example 8 to 12 carbon atoms.

Preferably when T₂ or T₃ is not the random statistical mixture of alkyl groups, T₂ or T₃ is hydrogen, branched alkyl of 3 to 12, preferably 4 to 8 carbon atoms or aralkyl of 7 to 9 carbon atoms, preferably alpha,alpha-dimethylbenzyl, but T₂ and T₃ are not both hydrogen at the same time. Also preferred are those compounds of formula I, wherein one of T₂ or T₃, in particular T₂, is ethyl or chlorine.

Preferred starting materials are mentioned under formulae (A) and (B) above. Preferred reaction conditions are stated when described in the process of the invention.

Under the instant process conditions the double bond in the alkene alkylating agent is isomerized along the carbon chain to give a random statistical mixture of moieties which can then be attached to the phenolic ring in the benzotriazole.

Illustrating with the alpha-olefin 1-octene, the random statistical mixture of octyl groups which would be included as T₂ or T₃ when T₂ or T₃ is octyl are, for example, 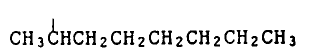 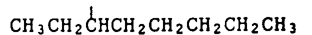 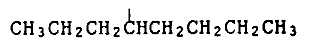 Thus T₂ or T₃ as octyl would lead to at least three isomers present in the mixture of benzotriazoles prepared.

Indeed, under the instant process conditions, the alkene being used in the instant process may fragment, dimerize or recombine in some fashion before reacting with the 2H-benzotriazole starting material. Such possibilities may in part account for the wide mixture of molecular species noted in the working Examples 1 - 11 where mass spectrographic analysis shows the presence of inter alia species with alkyl substituents having twice the number of carbon atoms as were present in the original alkene being used. In like manner, mass spectrographic analysis indicated some species with alkyl substituents having less than the number of carbon atoms in the alkene being used in the instant process or even having less than the number of carbon atoms in the alkyl groups in the original 2H-benzotriazole starting material used. In some cases, there were species having no alkyl substituents at all with complete dealkylation of the original 2H-benzotriazole having occurred. Inspection of the mass spectrographic analysis data shows that the combined amounts of all of these possible species enumerated in this paragraph are essentially minor components in the instant mixtures.

Although clearly the alkyl groups present in the instant mixtures are in an overwhelming degree present at the 3- and 5-positions (ortho and para positions) of the phenolic ring in the 2H-benzotriazoles of formula I, it cannot be ruled out that some substitution at the meta (4-) position or even in the benzo ring may not also occur in minor and inconsequential amounts.

In the case where the starting 2H-benzotriazole has a lower alkyl substituent on the benzo ring as R₁ or G, , said alkyl substituent is also unlikely to participate in the instant process since said benzo ring is quite deactivated and is not prone to direct alkylation and to participation in the instant process.

The alkenes useful in the instant process are any alkene whether straight or branched chain including alpha-olefins and alkenes having an internal double bond. During the instant alkylation process the double bond is isomerized along the carbon chain to give a random statistical mixture of alkenes which in turn produce for T₂ and T₃ a random statistical mixture of alkyl substituents.

Further contributing to the random statistical nature of the alkyl substituents for T₂ and T₃ is the fact that commercially available alkenes are often mixture of alkenes. That this mixture of isomeric radicals as T₂ or T₃ is critical to obtaining a liquid or non-crystalline product may be seen from the fact that, when the alkyl substitution is a specific isomer, solid crystalline products are obtained. For example, 2-(2-hydroxy-3,5-di-tert-octylphenyl)-2H-benzotriazole melts at 105 - 106 _{°} C.

The 2-(2-hydroxyphenyl)-2H-benzotriazole light absorbers are conveniently obtained by coupling an appropriately substituted phenol with an o-nitrophenyl diazonium salt to prepare an o-nitroazobenzen intermediate which is subsequently reduced and cyclized to the corresponding 2H-benzotriazole.

It is clear that any change in the nature of the substitution on the phenol moiety, for example for the purpose of modifying final 2H-benzotriazole properties, must be carried out on the phenol molecule itself before the conventional 2H-benzotriazole synthesis is begun. This requires one or more additional steps in the synthetic sequence for reach new 2H-benzotriazole product. Moreover, unavoidable side reactions occur during these steps which make it necessary to include at least one crystallization step in order to obtain a product of acceptable purity.

The above procedure is poorly adapted for the preparation of non-crystalline or liquid products where purification by crystallization is not possible.

Indeed the process described in U.S. Patent No. 4,129,521 discloses that, in order to obtain liquid products of acceptable purity, it is necessary to (1) vacuum distill the crude 2H-benzotriazole product, treat the once distille product with acetic anhydride to remove various undesirable impurities; carry out a second vacuum distillation on the acetylated mixture; blow the distillate with air at elevated temperature for many hours and finally distill the material for a third time under molecular distillation conditions. Only, then after these laborious and economically unattractive procedures is a liquid product useful as a light absorber obtained.

Clearly a better method of making liquid or non-crystalline 2H-benzotriazoles was needed since the conventional approach of preparing an alkylated phenol and then the benzotriazole from said phenol involves an almost impossible task of removing undesirable impurities from the benzotriazole in a practical manner.

The approach of alkylating a preformed 2H-benzotriazole was not believed promising since it was known that phenols substituted in the ortho position by a 2H-benzotriazolyl moiety are vastly deactivated in respect to electrophilic substitution (= alkylation) on the phenolic ring.

It was thus surprising that direct alkylation on the phenolic ring of preformed 2H-benzotriazoles could be carried out to give the desired mixed alkylated products in a facile and direct manner.

As described in U.S. Patent Nos. 4,587,346 and 4,675,352, the direct alkylation of 2-(2-hydroxy-5-methylphenyl)-2H-benzotriazole with an alpha-olefin or straight chain alkene; or with a branched alkene occurred in excellent conversions (over 90 %) of the preformed benzotriazole to alkylated products.

Since the 2-(2-hydroxy-5-methylphenyl)-2H-benzotriazole is ready substituted in the para position to the hydroxyl group, alkylation is perforce directed to the ortho position to the hydroxyl moiety to obtain a mixture of 2-(2-hydroxy-3-higher branched alkyl-5-methylphenyl)-2H-benzotriazoles.

The nature of the random statistical mixture of alkyl groups that are inserted into the 2H-benzotriazoles of formula I as T₂ or T₃ depends on which type of alkene is used for the alkylation.

The use of an alpha-olefin or, straight chain alkene leads to the insertion of branched secondary alkyl groups while the use of a branched alkene leads to branched alkyl groups having a multiplicity of alkyl branches along the main alkyl chain.

The starting materials of formula (A) and (B) are known or can be prepared by known methods, for example as described in the references cited in the introductory part.

Protection against damage caused by UV light is particularly important in photographic technology and especially in color photographic technology.

In order to protect the components (in particular dyes and couplers) present in a color photographic material as effectively as possible against destruction by ultraviolet light, UV absorbers are normally inserted into one or more of the upper layers of the material. This is effected as a rule by dissolving the UV-absorber in a high-boiling solvent and dispersing this solution, in the form of very fine droplets, in the appropriate coating solution. Since these droplets have a disadvantageous effect on the mechanical properties of the layer, and can "exude" if they are in the top layer of the material, it is important to keep the quantity of absorber solution as small as possible. This also makes it possible to produce thinner layers, which, in turn, offers advantages in processing (carry-over between baths and drying). It is therefore desirable to employ UV-absorbers which have as high a solubility as possible in the customary high-boiling solvents. The UV-absorbers of the state of the art, for example the stabilizers disclosed in Japanese Application Sho 54-95,233 do not, to a satisfactory extent, fulfil this requirement.

It has now been found that the mixtures according to this invention being liquid or non-crystalline can be used in color photographic material without the concomitant use of high-boiling solvents or with a very minimum amount thereof. Moreover, the instant mixtures are essentially non-volatile and do not exude.

A typical photographic composition comprises a paper support on which are coated one or more light-sensitive layers and a layer containing the ultraviolet light absorber in a binder so placed as to protect the layer or layers requiring protection.

It is known that ultraviolet radiation has a detrimental effect on photographic layers. Ultraviolet radiation in light sources used for exposure of photographic products sometimes produces undesired exposure of the layer or layers of a photographic element. This is especially true in photographic elements designed for use in color photography in which the emulsion has been sensitized to the longer wavelength regions and it is desirable to record only the rays of the visible spectrum.

Color photographs on multilayer photographic material, particularly those in which the dye images are formed in sensitive emulsion layers by color development, are susceptible to fading and discoloration by the action of ultraviolet radiation to which the photographs are subjected during viewing. The residual couplers contained in the emulsion layer after the formulation of the picture images may be attacked by ultraviolet radiation resulting in an undesirable stain in the finished photograph. The action of ultraviolet radiation on finished color photographs is particularly noticeable on positive prints on paper or other opaque support since this type of print is frequently viewed in daylight which has a high content of ultraviolet radiation. The dye-fading and discoloration effects appear to be caused primarily by those wavelengths of light close to the visual region of the spectrum, i.e., 300 - 400 nm.

It is known that silver halide photographic materials can be protected from ultraviolet radiation by incorporating nondiffusing ultraviolet absorbing compounds in the silver halide emulsion layers or in overlying colloid coatings.

A large number of ultraviolet absorbers have been proposed for this use. Ultraviolet absorbing compounds for photographic use must generally be colorless or nearly colorless, show good compatibility with the medium in which they are incorporated, be inert to other photographic addenda in the element and in the processing solution, must have good ultraviolet absorptivity and be stable to, ultraviolet radiation. Representative compounds for incorporation in photographic elements are described for example, in U.S. Patent No. 3,253,921.

Aromatic organic compounds such as ultraviolet absorbers, dye-forming couplers, antistain agents, filter dyes and the like to be effective must be nondiffusing and adequately distributed in highly dispersed form in the aqueous photographic gelatin layers.

This can be accomplished by a variety of chemical or physical techniques including the substitution of sulfonic acid or other solubilizing groups on the organic molecule; by use of a polar organic solvent imbibition procedures; or by solvent dispersion techniques.

The instant liquid or non-crystalline 2H-benzotriazole mixtures are extremely useful as ultraviolet absorbers in photographic gelatin layers. They exhibit desirable absorption characteristics in the ultraviolet region, i.e., maximum absorption in the near ultraviolet and sharp cut-off just outside the visible region, are essentially colorless, are readily dispersed or dissolved by either the solvent-dispersion or imbibition methods, and are photographically inert.

The instant compounds exhibit excellent compatibility characteristics in the gelatin layers of the photographic composition which lead to composition essentially without haze coupled with superior protection of the color dye images against the harmful effects of ultraviolet radiation. This combination of properties clearly distinguishes the instant benzotriazole light absorbers from the generic disclosure of U.S. Patent No. 3,253,921. These salubrious results are obtained when the instant benzotriazoles are incorporated directly into the gelatin layer or by the solvent dispersion technique.

An object of the invention is to provide novel photographic elements protected against the harmful effects of ultraviolet radiation by incorporation of ultraviolet absorbing materials. Another object is to provide photogrpahic color materials containing ultraviolet absorbers incorporasted in a highly stable form. A further object is to provide a non-diffusing ultraviolet absorber.

The invention relates further to stabilized organic material which is in the form of photographic material or is part of a photographic material, the photographic material containing, preferably in top layers, a mixture according to the invention. A preferred amount is 0.05 to 5 % by weight, relative to the photographic material without stabilizer.

When the instant mixtures are liquid, they are incorporated into a hydrophilic colloid by heating an aqueous solution of said hydrophilic colloid containing the liquid benzotriazole and an appropriate dispersion agent to a moderate temperature above the easy flow point of the instant benzotriazole, agitating the resulting mixture to obtain a fine dispersion of the benzotriazole in the colloid, and then cooling the mixture.

When the instant compounds are not liquid at room temperature, but are non-crystalline, the use of a minimum amount of high-boiling solvent to assist in getting the instant compound to flow is contemplated to achieve the above objects by the solvent dispersion technique to incorporate the instant compounds in aqueous hydrophilic colloid solutions for coating silver halide emulsion layers or associated hydrophilic colloid layers.

The preferred high-boiling solvents include di-n-butyl phthalate, benzyl phthalate, triphenyl phosphate, tri-o-cresyl phosphate, diphenyl mono-p-tert-butylphenyl phosphate, monophenyl di-p-tert-butylphenyl phosphate, diphenyl mono-o-chlorophenyl phosphate, monophenyl di-o-chlorophenyl phosphate, tri-p-tert-butylphenyl phosphate, tri-o-phenylphenyl phosphate, di-p-tert-butylphenyl mono(5-tert-butyl-2-phenylphenyl)-phosphate, etc.

The hydrophilic colloids or binders advantageously include gelatin, albumin, etc., cellulose derivatives, polyvinyl compounds, etc. The polymeric binders include polyvinyl alcohol or a hydrolyzed polyvinyl acetate; a far hydrolyzed cellulose ester such as cellulose acetate hydrolzyed to an acetyl content of 19 - 26 percent; a water-soluble ethanolamine cellulose acetate, a polyacrylamide having a combined acrylamide content of 30 - 60 percent and a specific viscosity of 0.25 - 1.5 or an imidized polyacrylamide of like acrylamide content and viscosity; a vinyl alcohol polymer containing urethane carboxylic acid groups; or containing cyanoacetyl groups such as the vinyl alcohol/vinyl cyanoacetate copolymer; or a polymeric material which results from polymerizing a protein or a saturated acylated protein with a monomer having a vinyl group.

The dispersion of an instant mixture in the binder material is coated over the light-sensitive layer of the photographic element. Where the photographic element is a material intended for use in color photography, the ultraviolet filter layer need not be an outer layer, but can be used as an interlayer, i.e., under the layer or layers not needing the protection and over the layer or layers needing protection. For example, in a multilayer material comprising three differentially sensitized layers, the red-sensitive layer being adjacent to the support, the green-sensitive layer being superimposed on the red-sensitive layer and the blue-sensitive layer being outermost with respect to the other light-sensitive layers, the ultraviolet filter layer can be placed between the blue and green-sensitive layers or between the green and red-sensitive layers. Similarly, in another photographic element in which the layers are reversed, that is, the blue-sensitive layer is coated over the support, and the green and red-sensitive layers are superposed over the blue-sensitive layer in that order, the ultraviolet filter layer can be over all three layers or between any two of the layers. Alternatively, the ultraviolet absorbing composition can be incorporated directly in the light-sensitive emulsion instead of, or in addition, being present in another layer. The amount of the ultraviolet absorbing material used can be varied, depending upon the effect desired and the use that will be made of the material.

The ultraviolet absorbing compositions are coated over a wide range of concentrations; usually they are coated in the range of from 20 to 300 mg of ultraviolet absorbing compound per ft² photographic element. A preferred range is from 75 to 160 mg/ft^{2 .} The optimum coating concentrations will depend upon the particular photographic element to be protected and the amount of protection desired. The optimum coating concentrations for a given photographic element can be determined by methods well known in the art.

Any photographic element may be advantageously protected according to the invention. These photographic elements may have as their suport any of the conventional support materials, such as firm supports, e.g., cellulose acetate, etc. opaque supports, such as white pigmented film, paper and the like.

The instant ultraviolet absorbing mixtures are characterized by their non-diffusibility in coated layers, good stability in the incorporating solvents and their good ultraviolet absorption. Ultraviolet absorbing layers containing the instant compounds incorporated according to the preferred methods of the invention have unexpectedly excellent stability upon prolonged exposure to ultraviolet radiation which makes them ideally suited for protecting photographic elements, particularly dye images in color materials.

The instant liquid benzotriazole mixtures may be used advantageously in photographic elements with other liquid ultraviolet absorbers (UVA) such as 5-chloro-2-[2-hydroxy-3-tert-butyl-5-(2-octyloxyethyl)-phenyl]-2H-benzotriazole.

The instant liquid benzotriazoles are also useful as solvents for other solid UVA materials or for other components in a silver halide photographic element when used alone or in combination with common photographic oils as described in European Patent Application Nos. 84,692 and 84,694.

Such other components include
- yellow, magenta and cyan couplers
- DIR couplers, black couplers, colorless couplers
- chromogenic coupler stabilizers
- chromogenic dye stabilizers
- accutance dyes, antihalation dyes, dye-bleach dyes
- formaldehyde scavengers
- sensitizing dyes
- optical brightening agents
- oxidized developer scavengers
- compounds which release diffuseable dyes on development
- electron transfer agents

Examples of other UVA materials which may be used in combination with the instant compounds include
1. Benzophenones, for example 2,4-dihydroxy-benzophenone, 2-hydroxy-4-ethoxy-benzophenone, 2,2'-dihydroxy-4-methoxy-benzophenone, 2-hydroxy-4-n-octoxy-benzophenone, 2-hydroxy-4-isooctoxy-benzophenone, 2-hydroxy-4-dodecyloxy-benzophenone.
2. Benzotriazoles, for example 2-(2-hydroxy-5-methylphenyl)-benzotriazole, 2-(2-hydroxy-3,5-di-t-butylphenyl)-benzotriazole, 2-(2-hydroxy-3-t-butyl-5-ethylphenyl)-5-chlorobenzotriazole, 2-(2-hydroxy-3,5-di-t-butylphenyl)-5-chlorobenzotriazole, 2-(2-hydroxy-3,5-di-tert-amylphenyl)-benzotriazole, 2-(2-hydroxy-3-s-butyl-5-t-butylphenyl)-benzotriazole, 2-(2-hydroxy-5-t-butylphenyl)-benzotriazole, 2-(2-hydroxy-5-t-octylphenyl)-benzotriazole, a mixture of 50 % of 2-[(2-hydroxy-3-t-butyl-5-((2"-n-octoxy-carbonyl)-ethyl)-phenyl]-5-chlorobenzotriazole and 50 % of 2-[(2-hydroxy-3-t-butyl-5-((2"-ethylhexyloxy)carbonyl)ethyl)-phenyl]-5-chlorobenzotriazole, 2-[2-hydroxy-3,5-di-(alpha,alpha-dimethylbenzyl)-phenyl]-benzotriazole, reaction products of 2-[(2-hydroxy-3-t-butyl-5-((2-methoxycarbonyl)-ethyl)phenyl]-chlorobenzotriazole with polyethylene glycols, in particular with polyethylene glycol 300.
3. Benzylidene malonates, for example methyl 2-carboxymethyl-3-(4'-methoxyphenyl)-acrylate.
4. Salicylates, for example p-octylphenyl salicylate, phenyl salicylate, t-butylphenyl salicylate.
5. Monobenzoates, for example resorcinol monobenzoate, 3,5-di-t-butyl-4-hydroxybenzoic acid hexadecyl ester.
6. Oxamides, for example 5-t-butyl-2-ethoxy-2'2'-ethyloxanilide, 2-ethoxy-2'-ethyloxanilide.
7. 5-dialkylamino-2,4-pentadienoic acid esters, for example 5-diethylamino-2-phenylsulphonyl-2,4-pen- tadienoic acid hendecylester.
8. 5-dialkylamino-2-cyano-2,4-pentadiene nitriles, for example 5-dihexylamino-2-cyano-2,4-pentadiene nitrile.
9. 2,4-di-t-butylphenyl 3,5-di-t-butyl-4-hydroxy-benzoate, 3,5-ditertiary-butyl-p-hydroxy-benzoic acid, di-(1,2,2,6,6-pentamethyl-4-piperidinyl)-butyl (3',5'-di-t-butyl-4-hydroxybenzyl)malonate, bis(1,2,6,6-tetramethyl-4-piperidinyl)sebacate, bis(1,2,2,6,6-pentamethyl-4-piperidyl)sebacate, butane tetracarboxylic acid tetra(2,2,6,6-tetramethyl-4-piperidinyl)ester.

The instant compounds may also be used in cyan layers together with either phenol, naphthol or 2,5- diacylaminophenol couplers or mixtures of these couplers to prevent image fading and discoloration.

The use of known benzotriazoles in such systems is described in Japanese Kokai Sho 58-221844 and 59-46646.

In general, the mixtures of this invention are effective light stabilizers in a wide range of organic materials, in particular of organic polymers. Polymers which can be stabilized include:
1. Polymers of monoolefins and diolefins, for example polypropylene, polyisobutylene, polybutene-1, polymethylpentene-1, polyisoprene or polybutadiene, as well as polymers of cycloolefins, for instance of cyclopentene or norbornene, polyethylene (which optionally can be crosslinked), for example high density polyethylene (HDPE), low density polyethylene (LDPE) and linear low density polyethylene (LLDPE).
2. Mixtures of the polymers mentioned under 1), for example mixtures of polypropylene with polyisobutylene, polypropylene with polyethylene (for example PP/HDPE, PP/LDPE) and mixtures of different types of polyethylene (for example LDPE/HDPE).
3. Copolymers of monoolefines and diolefines with each other or with other vinyl monomers, such as, for example, ethylene/propylene, linear low density polyethylene (LLDPE) and its mixtures with low density polyethylene (LDPE), propylene/butene-1, ethylene/hexene, ethylene/ethylpentene, ethylene/heptene, ethylene/octene, propylene/isobutylene, ethylene/butene-1, propylene/butadiene, isobutylene/isoprene, ethylene/alkyl acrylates, ethylene/alkyl methacrylates, ethylene/vinyl acetate or ethylene/ acrylic acid copolymers and their salts (ionomers) and terpolymers of ethylene with propylene and a diene, such as hexadiene, dicyclopentadiene or ethylidene-norbornene; as well as mixtures of such copolymers and their mixtures with polymers mentioned in 1) above, for example polypropylene/ethylene-propylene- copolymers, LDPE/EVA, LDPE/EAA, LLDPE/EVA and LLDPE/EAA.
   3a. Hydrocarbon resins (for example Cₛ-Cₛ) and hydrogenated modifications thereof (for example tackyfiers).
4. Polystyrene, poly-(p-methylstyrene), poly-(a-methylstyrene).
5. Copolymers of styrene or a-methylstyrene with dienes or acrylic derivatives, such as, for example, styrene/butadiene, styrene/ acrylonitrile, styrene/alkyl methacrylate, styrene/maleic anhydride, sty- rene/butadiene/ethyl acrylate, styrene/acrylonitrile/methyl acrylate; mixtures of high impact strength from styrene copolymers and another polymer, such as, for example, from a polyacrylate, a diene polymer or an ethylene/propylene/diene terpolymer; and block copolymers of styrene, such as, for example, styrene/butadiene/ styrene, styrene/ isoprene/styrene, styrene/ethylene/butylene/ styrene or styrene/ ethylene/propylene/styrene.
6. Graft copolymers of styrene or a-methylstyrene such as, for example, styrene on polybutadiene, styrene on polybutadiene-styrene or polybutadiene-acrylonitrile; styrene and acrylonitrile (or methacrylonitrile) on polybutadiene; styrene and maleic anhydride or maleimide on polybutadiene; styrene, acrylonitrile and maleic anhydride or maleimide on polybutadiene; styrene, acrylonitrile and methyl methacrylate on polybutadiene, styrene and alkyl acrylates or methacrylates on polybutadiene, styrene and acrylonitrile on ethylene/propylene/diene terpolymers, styrene and acrylonitrile on polyacrylates or polymethacrylates, styrene and acrylonitrile on acrylate/butadiene copolymers, as well as mixtures thereof with the copolymers listed under 5), for instance the copolymer mixtures known as ABS-, MBS-, ASA- or AES-polymers.
7. Halogen-containing polymers, such as polychloroprene, chlorinated rubbers, chlorinated or sulfochlorinated polyethylene, epichlorohydrin homo- and copolymers, polymers from halogen-containing vinyl compounds,as for example, polyvinylchloride, polyvinylidene chloride, polyvinyl fluoride, polyvinylidene fluoride, as well as copolymers thereof, as for example, vinyl chloride/vinylidene chloride, vinyl chloride/vinyl acetate or vinylidene chloride/vinyl acetate copolymers.
8. Polymers which are derived from a,;8-unsaturated acids and derivatives thereof, such as polyacrylates and polymethacrylates, polyacrylamide and polyacrylonitrile.
9. Copolymers from the monomers mentioned under 8) with each other or with other unsaturated monomers, such as, for instance, acrylonitrile/butadiene, acrylonitrile/alkyl acrylate, acrylonitrile/ alkoxyalkyl acrylate or acrylonitrile/vinyl halogenide copolymers or acrylonitrile/alkyl methacrylate/butadiene terpolymers.
10. Polymers which are derived from unsaturated alcohols and amines, or acyl derivatives thereof or acetals thereof, such as polyvinyl alcohol, polyvinyl acetate, polyvinyl stearate, polyvinyl benzoate, polyvinyl maleate, polyvinyl butyral, polyallyl phthalate or polyallylmelamine; as well as their copolymers with olefins mentioned in 1) above.
11. Homopolymers and copolymers of cyclic ethers, such as polyalkylene glycols, polyethylene oxide, polypropylene oxide or copolymers thereof with bis-glycidyl ethers.
12. Polyacetals, such as polyoxymethylene and those polyoxymethylenes which contain ethylene oxide as a comonomer; polyacetals modified with thermoplastic polyurethanes, acrylates or MBS.
13. Polyphenylene oxides and sulfides, and mixtures of polyphenylene oxides with polystyrene or polyamides.
14. Polyurethanes which are derived from polyethers, polyesters or polybutadienes with terminal hydroxyl groups on the one side and aliphatic or aromatic polyisocyanates on the other side, as well as precursors thereof (polyisocyanates, polyols or prepolymers).
15. Polyamides and copolyamides which are derived from diamines and dicarboxylic acids and/or from aminocarboxylic acids or the corresponding lactams, such as polyamide 4, polyamide 6, polyamide 6/6, 6/10, 6/9, 6/12 and 4/6, polyamide 11, polyamide 12, aromatic polyamides obtained by condensation of m-xylene diamine and adipic acid; polyamides prepared from hexamethylenediamine and isophthalic or/and terephthalic acid and optionally an elastomer as modifier, for example poly-2,4,4,- trimethylhexamethylene terephthalamide or poly-m-phenylene isophthalamide. Further copolymers of the aforementioned polyamides with polyolefins, olefin copolymers, ionomers or chemically bonded or grafted elastomers; or with polyethers, such as for instance, with polyethylene glycols, polypropylene glycols or polytetramethylene glycols. Polyamides or copolyamides modified with EPDM or ABS. Polyamides condensed during processing (RIM-polyamide systems).
16. Polyureas, polyimides and polyamide-imides.
17. Polyesters which are derived from dicarboxylic acids and diols and/or from hydroxycarboxylic acids or the corresponding lactones, such as polyethylene terephthalate, polybutylene terephthalate, poly-1,4-di-methylolcyclohexane terephthalate, poly-[2,2,-(4-hydroxyphenyl)-propane] terephthalate and polyhydroxybenzoates as well as block-copolyether-esters derived from polyethers having hydroxyl end groups.
18. Polycarbonates and polyester-carbonates.
19. Polysulfones, polyether-sulfones and polyether-ketones.
20. Crosslinked polymers which are derived from aldehydes on the one hand and phenols, ureas and melamines on the other hand, such as phenol/formaldehyde resins, urea/formaldehyde resins and melamine/formaldehyde resins.
21. Drying and non-drying alkyd resins.
22. Unsaturated polyester resins which are derived from copolyesters of saturated and unsaturated dicarboxylic acids with polyhydric alcohols and vinyl compounds as crosslinking agents, and also halogen-containing modifications thereof of low inflammability.
23. Thermosetting acrylic resins, derived from substituted acrylic esters, such as epoxy-acrylates, urethane-acrylates or polyester-acrylates.
24. Alkyd resins, polyester resins or acrylate resins in admixture with melamine resins, urea resins, polyisocyanates or epoxide resins as crosslinking agents.
25. Crosslinked epoxide resins which are derived from polyepoxides, for example from bis-glycidyl ethers or from cycloaliphatic diepoxides.
26. Natural polymers, such as cellulose, rubber, gelatine and derivatives thereof which are chemically modified in a polymer-homologous manner, such as cellulose acetates, cellulose propionates and cellulose butyrates, or the cellulose ethers, such as methylcellulose; resins and their derivatives.
27. Mixtures of polymers as mentioned above, for example PP/EPDM, Polyamide 6/EPDM or ABS, PVC/EVA, PVC/ABS, PVC/MBS, PC/ABS, PBTP/ABS, PC/ASA, PC/PBT, PVC/CPE, PVC/acrylates, POM/thermoplastic PUR, PC/thermoplastic PUR, POM/acrylate, POM/MBS, PPE/HIPS, PPE/PA 6.6 and copolymers, PA/HDPE, PA/PP, PA/PPE.
28. Naturally occurring and synthetic organic materials which are pure monomeric compounds or mixtures of such compounds, for example mineral oils, animal and vegetable fats, oil and waxes, or oils, fats and waxes based on synthetic esters (e.g. phthalates, adipates, phosphates or trimellithates) and also mixtures of synthetic esters with mineral oils in any weight ratios, which materials may be used as plasticizer for polymers or as textile spinning oils, as well as aqueous emulsions of such materials.

Therefore, the present invention consists also in the use of the instant mixtures as stabilizers, in particular light stabilizers, for organic materials. It further provides stabilized compositions which comprise
(a) an organic material, and
(b) a mixture as defined in claim 10 or 11, as stabilizer.

The organic material is preferably an organic polymer, especially a synthetic polymer.

The stabilizing of polyolefins, styrene polymers, polyacrylates, polyamides, polyurethanes, halogen containing vinyl polymers, alkyd resins, thermoset acrylic resins, and epoxy resins is of particular importance, and the instant benzotriazole mixtures are outstandingly suitable for this purpose. Examples of such polymers are high density and low density polethylene, polypropylene, ethylene/propylene copolymers, polystyrene, styrene block copolymers, halogen containig vinyl polymers, linear (= thermoplastic) and crosslinked (= thermoset) polyacrylates and polyurethanes, alkyd resins and epoxy resins in the form of coatings, lacquers, filaments, films, sheets, adhesives, elastomers, foams or shaped articles.

The instant stabilizers are added to the substrates in an effective amount, for example in a concentration of 0.05 to 10 % by weight, calculated relative to the material to be stabilized. Preferably, 0.1 to 5 % by weight of the stabilizer calculated relative to the material to be stabilized, is incorporated into the latter.

Incorporation can be effected after polymerization, for example by mixing the stabilizer mixtures and, if desired, further additives into the melt by the methods customary in the art, before or during shaping, or by applying the dissolved or dispersed compounds to the polymer, with subsequent evaporation of the solvent if necessary.

The stabilizers can also be added to the substrates to be stabilized in the form of a master batch which contains these compounds, for example in a concentration of 2.5 to 25 % by weight.

Although the mixures of the invention provide excellent light stability, the compounds of this invention are often combined with other stabilizers, even other light stabilizers, in the preparation of stabilized compositions. The stabilizers may be used with phenolic antioxidants, pigments, colorants or dyes, light stabilizers such as hindered amines, metal deactivators, etc.

The mixtures according to this invention may readily be incoporated into the organic substrates by conventional techniques, at any convenient stage prior to the manufacture of shaped articles therefrom. For example, the stabilizer may be mixed with the dry polymer, or a suspension, solution or emulsion of the stabilizer may be mixed with a solution, suspension, or emulsion of the polymer. The stabilized compositions, in particular polymer compositions, of the invention may optionally also contain various conventional additives, for example in an amount of about 0.05 to about 10 %, preferably from about 0.1 to about 5 %, by weight, such as the following, particulary phenolic antioxidants or lightstabilizers, or mixtures thereof:
1. Antioxidants
   1.1. Alkylated monophenols, for example 2,6-di-tert-butyl-4-methylphenol, 2-tert-butyl-4,6-dimethylphenol, 2,6-di-tert-butyl-4-ethylphenol, 2,6-di-tert-butyl-4-n-butylphenol, 2,6-di-tert-butyl-4-isobutylphenol, 2,6-dicyclopentyl-4-methylphenol, 2-(a-methylcyclohexyl)-4,6-d i methyl phenol, 2,6-dioctadecyl-4-methylphenol, 2,4,6-tricyclohexylphenol, 2,6-di-tert-butyl-4-methoxymethylphenol, 2,6-di-nonyl-4-methylphenol.
   1.2. Alkylated hydroquinones,for example 2,6-di-tert-butyl-4-methoxyphenol, 2,5-di-tert-butylhydroquinone, 2,5-di-tert-amylhydroquinone, 2,6-diphenyl-4-octadecyloxyphenol.
   1.3. Hydroxylated thiodiphenyl ethers, for example 2,2'-thiobis(6-tert-butyl-4-methylphenol), 2,2'-thiobis(4-octylphenol), 4,4'-thiobis(6-tert-butyl-3-methylphenol), 4,4'-thiobis(6-tert-butyl-2-methylphenol).
   1.4. Alkylidenebisphenols, for example 2,2'-methylenebis(6-tert-butyl-4-methylphenol), 2,2'-methylenebis(6-tert-butyl-4-ethylphenol), 2,2'-methylenebis[4-methyl-6-(a-methylcyclohexyl)phenol]-,2,2'-methylenebis(4-methyl-6-cyclohexylphenol), 2,2'-methylenebis(6-nonyl-4-methylphenol), 2,2'-methylenebis(4,6-di-tert-butylphenol), 2,2'-ethylidenebis(4,6-di-tert-butylphenol), 2,2'-ethylidenebis(6-tert-butyl-4-isobutylphenol), 2,2'-methylenebis[6-(a-methylbenzyl)-4-nonylphenol], 2,2'-methylenebis[6-(α,α-dimethylbenzyl)-4-nonylphenol], 4,4'-methylenebis(2,6-di-tert-butylphenol), 4,4'-methylenebis(6-tert-butyl-2-methylphenol), 1,1-bis(5-tert-butyl-4-hydroxy-2-methylphenyl)butane, 2,6-bis(3-tert-butyl-5-methyl-2-hydroxybenzyl)-4-methylphenol, 1,1,3-tris(5-tert-butyl-4-hydroxy-2-methylphenyl)butane, 1,1- bis(5-tert-butyl-4-hydroxy-2-methylphenyl)-3-n-dodecylmercaptobutane, ethylene glycol bis[3,3-bis(3'-tert-butyl-4'-hydroxyphenyl)butyrate],bis(3-tert-butyl-4-hydroxy-5-methylphenyl)dicydopentadiene, bis-[2-(3'-tert-butyl-2'-hydroxy-5'-methylbenzyl)-6-tert-butyl-4methylphenyl] terephthalate.
   1.5. Benzyl compounds, for example 1,3,5-tris(3,5-di-tert-butyl-4-hydroxybenzyl)-2,4,6-trimethylbenzene, bis(3,5-di-tert-butyl-4-hydroxybenzyl) sulfide, isooctyl 3,5-di-tert-butyl-4-hydroxybenzylmercapto- acetate, bis(4-tert-butyl-3-hydroxy-2,6-dimethylbenzyl) dithiolterephthalate, 1,3,5-tris(3,5-di-tert-butyl-4-hydroxybenzyl) isocyanurate, 1,3,5-tris(4-tert-butyl-3-hydroxy-2,6-dimethylbenzyl) isocyanurate, dioctadecyl 3,5-di-tert-butyl-4-hydroxybenzylphosphonate, calcium salt of monoethyl 3,5-di-tert-butyl-4-hydroxybenzylphosphonate, 1,3,5-tris-(3,5-dicyclohexyl-4-hydroxybenzyl)isocyanurate.
   1.6. Acylaminophenols, for example lauric acid 4-hydroxyanilide, stearic acid 4-hydroxyanilide, 2,4-bis-(octylmercapto)-6-(3,5-di-tert-butyl-4-hydroxyanilino)-s-triazine, octyl N-(3,5-di-tert-butyl-4-hydroxyphenyl)carbamate.
   1.7. Esters of β-(3,5-di-tert-butyl-4-hydroxyphenyl)propionic acid with mono- or polyhydric alcohols, e.g. with methanol, diethylene glycol, octadecanol, triethylene glycol, 1,6-hexanediol, pentaerythritol, neopentyl glycol, tris(hydroxyethyl) isocyanurate, thiodiethylene glycol, N,N'-bis(hydroxyethyl)oxalic acid diamide.
   1.8. Esters of β-(5-tert-butyl-4-hydroxy-3-methylphenyl)propionic acid with mono- or polyhydric alcohols, e.g. with methanol, diethylene glycol, octadecanol, triethylene glycol, 1,6-hexanediol, pentaerythritol, neopentyl glycol, tris(hydroxyethyl) isocyanurate, thiodiethylene glycol, N,N'-bis-(hydroxyethyl)oxalic acid diamide.
   1.9. Esters of β-(3,5-dicyclohexyl-4-hydroxyphenyl)propionic acid with mono- or polyhydric alcohols, e.g. with methanol, diethylene glycol, octadecanol, triethylene glycol, 1,6-hexanediol, pentaerythritol, neopentyl glycol, tris(hydroxyethyl) isocyanurate, thiodiethylene glycol, N,N'-bis(hydroxyethyl)oxalic acid diamide.
   1.10. Amides of β-(3,5-di-tert-butyl-4-hydroxyphenyl)propionic acid e.g. N,N'-bis(3,5-di-tert-butyl-4-hydroxyphenylpropionyl)hexamethylene-diamine, N,N'-bis(3,5-di-tert-butyl-4-hydroxyphenylpropionyl)-trimethylenediamine, N,N'-bis(3,5-di-tert-butyl-4-hydroxyphenylpropionyl)hydrazine.
2. UV absorbers and light stabilisers
   2.1. 2-(2'-Hydroxyphenyl)benzotriazoles, for example the 5'-methyl, 3',5'-di-tert-butyI, 5'-tert-butyl, 5'-(1,1,3,3-tetramethylbutyl), 5-chloro-3' ,5'-di-tert-butyl, 5-chloro-3'-tert-butyl-5'-methyl, 3'-sec-butyl-5'-tert-butyl, 4'-octoxy, 3' ,5'-di-tert-amyl and 3',5'-bis(a,a-dimethylbenzyl) derivatives.
   2.2. 2-Hydroxybenzophenones, for example the 4-hydroxy, 4-methoxy, 4-octoxy, 4-decyloxy, 4-dodecyloxy, 4-benzyloxy, 4,2',4'-trihydroxy and 2'-hydroxy-4,4'-dimethoxy derivatives.
   2.3. Esters of substituted and unsubstituted benzoic acids, for example, 4-tert-butylphenyl salicylate, phenyl salicylate, octylphenyl salicylate, dibenzoylresorcinol, bis(4-tert-butylbenzoyl)-resorcinol, benzoylresorcinol, 2,4-di-tert-butylphenyl 3,5-di-tert-butyl-4-hydroxybenzoate and hexadecyl 3,5-di-tert-butyl-4-hydroxybenzoate.
   2.4. Acrylates, for example ethyl α-cyano-β,β-diphenylacrylate, isooctyl α-cyano-β,β-diphenylacrylate, methyl a-carbomethoxycinnamate, methyl α-cyano-β-methyl-p-methoxy-cinnamate, butyl α-cyano-β-methyl-p-methoxycinnamate, methyl a-carbomethoxy-p-methoxycinnamate and N-(β-carbomethoxy-β-cyanovinyl)-2-methylindoline.
   2.5. Nickel compounds, for example nickel complexes of 2,2'-thio-bis-[4-(1,1,3,3-tetramethylbutyl)-phenol], such as the 1:1 or 1:2 complex, with or without additional ligands such as n-butylamine, triethanolamine or N-cyclohexyldiethanolamine, nickel dibutyldithiocarbamate, nickel salts of 4-hydroxy-3,5-di-tert-butylbenzyl-phosphonic acid monoalkyl esters, e.g. of the methyl or ethyl ester, nickel complexes of ketoximes, e.g. of 2-hydroxy-4-methyl-phenyl undecyl ketoxime, nickel complexes of 1-phenyl-4-lauroyl-5-hydroxypyrazole, with or without additional ligands.
   2.6. Sterically hindered amines, for example bis(2,2,6,6-tetramethylpiperidyl) sebacate, bis(1,2,2,6,6-pentamethylpiperidyl) sebacate, bis(1,2,2,6,6-pentamethylpiperidyl) n-butyl-3,5-di-tert-butyl-4-hydrox- ybenzylmalonate, the condensation product of 1-(2-hydroxyethyl)-2,2,6,6-tetramethyl-4-hydrox- ypiperidine and succinic acid, the condensation product of N,N'-bis(2,2,6,6-tetramethyl-4-piperidyl)-hexamethylenediamine and 4-tert-octylamino-2,6-dichloro-1,3,5-triazine, tris(2,2,6,6-tetramethyl-4-piperidyl) nitrilotriacetate, tetrakis(2,2,6,6-tetramethyl-4-piperidyl)-1 ,2,3,4-butane-tetracarboxylate, 1,1'-(1,2-ethanediyl)bis(3,3,5,5-tetramethylpiperazinone).
   2.7. Oxalic acid diamides, for example 4,4'-dioctyloxyoxanilide, 2,2'-di-octyloxy-5,5'-di-tert-butylox- anilide, 2,2'-didodecyloxy-5,5'-di-tert-butyloxanilide, 2-ethoxy-2'-ethyloxanilide, N,N'-bis(3-dimethylaminopropyl)oxalamide, 2-ethoxy-5-tert-butyl-2'-ethyloxanilide and its mixture with 2-ethoxy-2'-ethyl-5,4'-di-tert-butyloxanilide and mixtures of ortho-and para-methoxy-disubstituted oxanilides and mixtures of o-and p-ethoxy-disubstituted oxanilides.
   2.8. 2-(2-Hydroxyphenyl)-1,3,5-triazines, for example 2,4,6-tris(2-hydroxy-4-octyloxyphenyl)-1,3,5-triazine, 2-(2-hydroxy-4-octyloxyphenyl)-4,6-bis(2,4-dimethylphenyl)-1,3,5-triazine, 2-(2,4-dihydroxyphenyl)-4,6-bis(2,4-dimethylphenyl)-1,3,5-triazine, 2,4-bis(2-hydroxy-4-propyloxyphenyl)-6-(2,4-dimethylphenyl)-1,3,5-triazine, 2-(2-hydroxy-4-octyloxyphenyl)-4,6-bis(4-methylphenyl)-1,3,5-triazine, 2-(2-hydroxy-4-dodecyloxyphenyl)-4,6-bis(2,4-dimethylphenyl)-1,3,5-triazine.
3. Metal deactivators, for example N,N'-diphenyloxalic acid diamide, N-salicylal-N'-salicyloylhydrazine, N,N'-bis(salicyloyl)hydrazine, N,N'-bis(3,5-di-tert-butyl-4-hydroxyphenylpropionyl)hydrazine, 3- salicyloylamino-1,2,4-triazole, bis(benzylidene)oxalodihydrazide.
4. Phosphites and phosphonites, for example triphenyl phosphite, diphenyl alkyl phosphites, phenyl dialkyl phosphites, tris(nonylphenyl) phosphite, trilauryl phosphite, trioctadecyl phosphite, distearyl pentaerythritol diphosphite, tris(2,4-di-tert-butylphenyl) phosphite, diisodecyl pentaerythritol diphosphite, bis-(2,4-di-tertbutylphenyl) pentaerythritol diphosphite, tristearyl sorbitol triphosphite, tetrakis(2,4-di-tert-butylphenyl) 4,4'-biphenylene diphosphonite, 3,9-bis(2,4-di-tert-butylphenoxy)-2,4,8,1 0-tetraoxa-3,9- diphosphaspiro[5.5]undecane.
5. Peroxide scavengers, for example esters of Q-thiodipropionic acid, for example the lauryl, stearyl, myristyl or tridecyl esters, mercaptobenzimidazole or the zinc salt of 2-mercaptobenzimidazole, zinc dibutyldithiocarbamate, dioctadecyl disulfide, pentaerythritol tetrakis(Q-dodecylmercapto)propionate.
6. Polyamide stabilisers, for example, copper salts in combination with iodides and/or phosphorus compounds and salts of divalent manganese.
7. Basic co-stabilisers, for example, melamine, polyvinylpyrrolidone, dicyandiamide, triallyl cyanurate, urea derivatives, hydrazine derivatives, amines, polyamides, polyurethanes, alkali metal salts and alkaline earth metal salts of higher fatty acids for example Ca stearate, Zn stearate, Mg stearate, Na ricinoleate and K palmitate, antimony pyrocatecholate or zinc pyrocatecholate.
8. Nucleating agents, for example, 4-tert.butyl-benzoic acid, adipic acid, diphenylacetic acid.
9. Fillers and reinforcing agents, for example, calcium carbonate, silicates, glass fibres, asbestos, talc, kaolin, mica, barium sulfate, metal oxides and hydroxydes, carbon black, graphite.
10. Other additives, for example, plasticisers, lubricants, emulsifiers, pigments, optical brighteners, flameproofing agents, antistatic agents and blowing agents.

Other additives that can be incorporated in the stabilized compositions are thiosynergists such as dilauryl thiodipropionate, lubricants such as stearyl alcohol, fillers, asbestos, kaolin, talc, glass fibers, pigments, optical brighteners, flameproofing agents and antistatic agents.

The following examples are presented for the purpose of further illustration only and are not to be construed to limit the nature or scope of the instant invention in any manner whatsoever. Parts and percentages in this specification are by weight where not otherwise stated.

Examples 1 - 11: In a flask fitted with a nitrogen blanket, stirrer, reflux condenser and addition funnel, 0.5 mole of olefin, 0.5 mole of the 2H-benzotriazole and 0.5 mole of 98 % methanesulfonic acid are heated to 160°C and held at this temperature for a period of four hours during which time an additional 1.5 moles of olefin are added to the reaction mixture.

The mixture is then cooled to 20 ° C. The lower methanesulfonic acid layer is separated off and the upper product layer is washed once with 1000 ml of 2 % sodium carbonate solution and finally dried over anhydrous magnesium sulfate.

The dried organic solution is vacuum stripped at 170°C/0.5 mm Hg to remove the unreacted olefin. The residual material is then vacuum distilled on a Kugelrohr column to give a fraction boiling at about 170 - 180 ° C/0.1 mm Hg which includes some hydrocarbon material and the desired products boiling at about 180 - 210 ° C/0.05 mm Hg. The desired products are in every case viscous yellow liquids.

The products prepared by the method described above are analyzed by chemical ionization mass spectrometry using a direct probe inlet. Control samples of the various starting materials confirm that the observed product distributions are real and are not artifacts due to fragmentation inside the mass spectrometer.

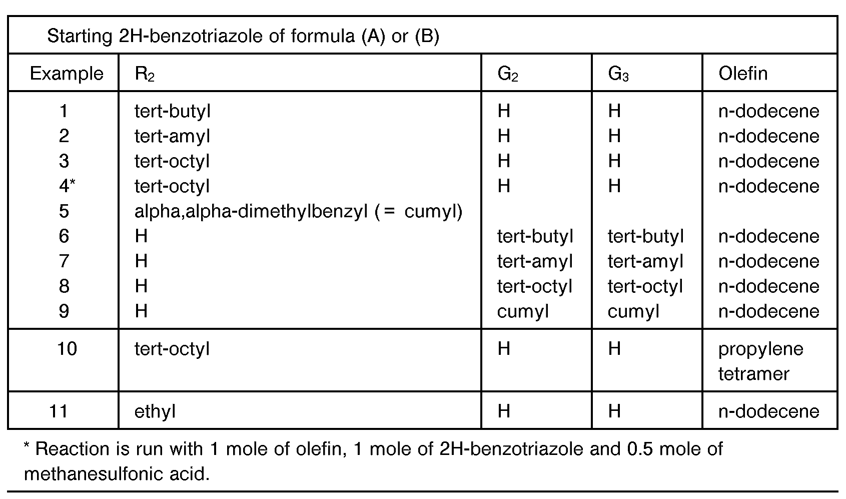

The mass spectrometer analyses of the reaction products obtained from Examples 1 - 11 are given on Tables 1 - 11 below.

Tables 1 - 5 and 10 - 11 give the results of reacting an olefin with a 2-(2-hydroxy-5-alkyl(or cumyl)-phenyl)-2H-benzotriazole. Tables 6 - 9 show the results of reacting an olefin with a 2-(2-hydroxy-3,5-di-tert-alkyl(or cumyl)phenyl)-2H-benzotriazole.

In Tables 1 - 5 and 10 - 11, the "normal" products which would be obtained by olefin addition to the 2H-benzotriazole without any displacement, fragmentation or other reaction are asterisked. Such products amount to much less than 50 % of the product mixture.

In Tables 6 - 9, the products obtained by an alkyl exchange reaction where one alkyl (or cumyl) is replaced by a dodecyl moiety are asterisked. Such products occur in the 10 - 36 % of the final product mixture.

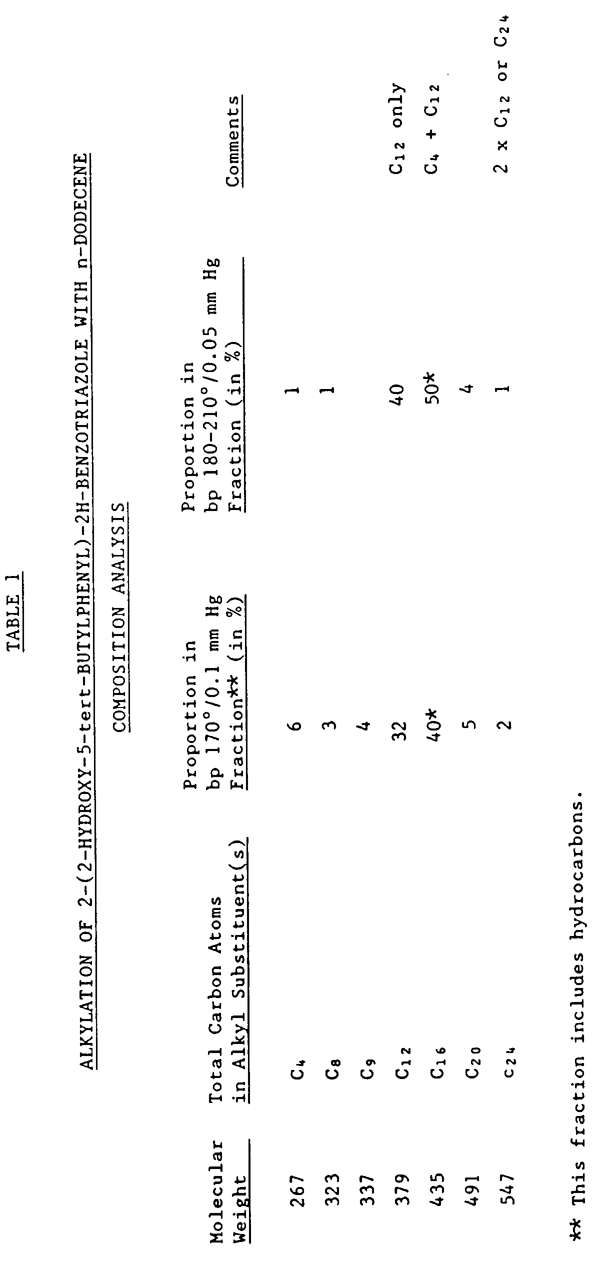

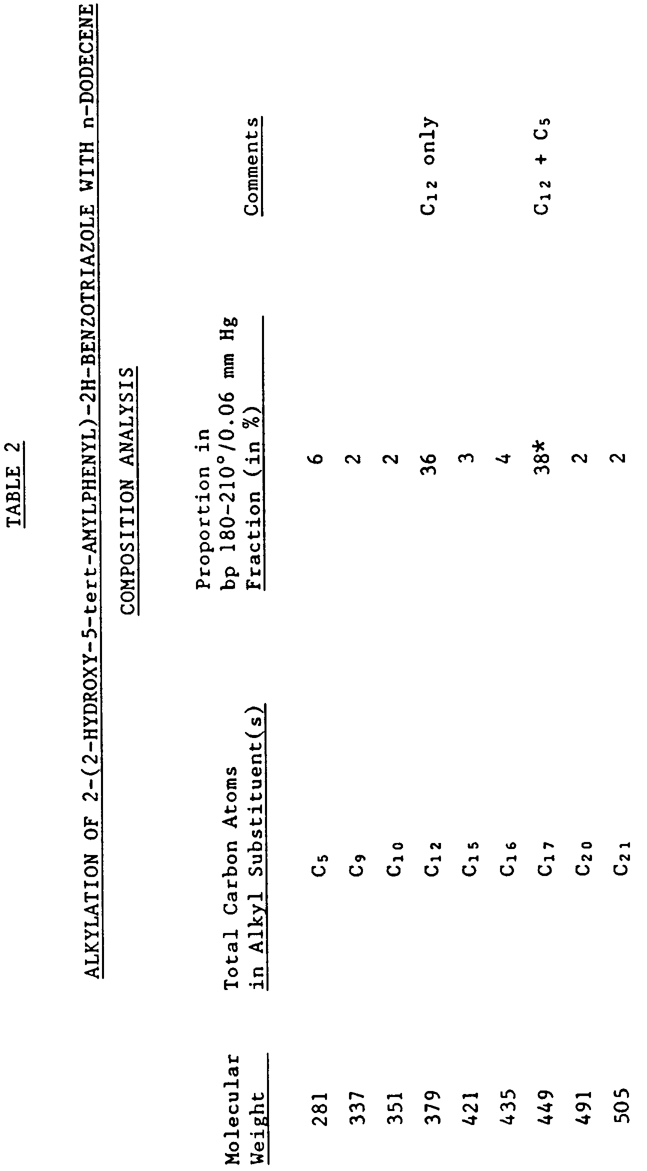

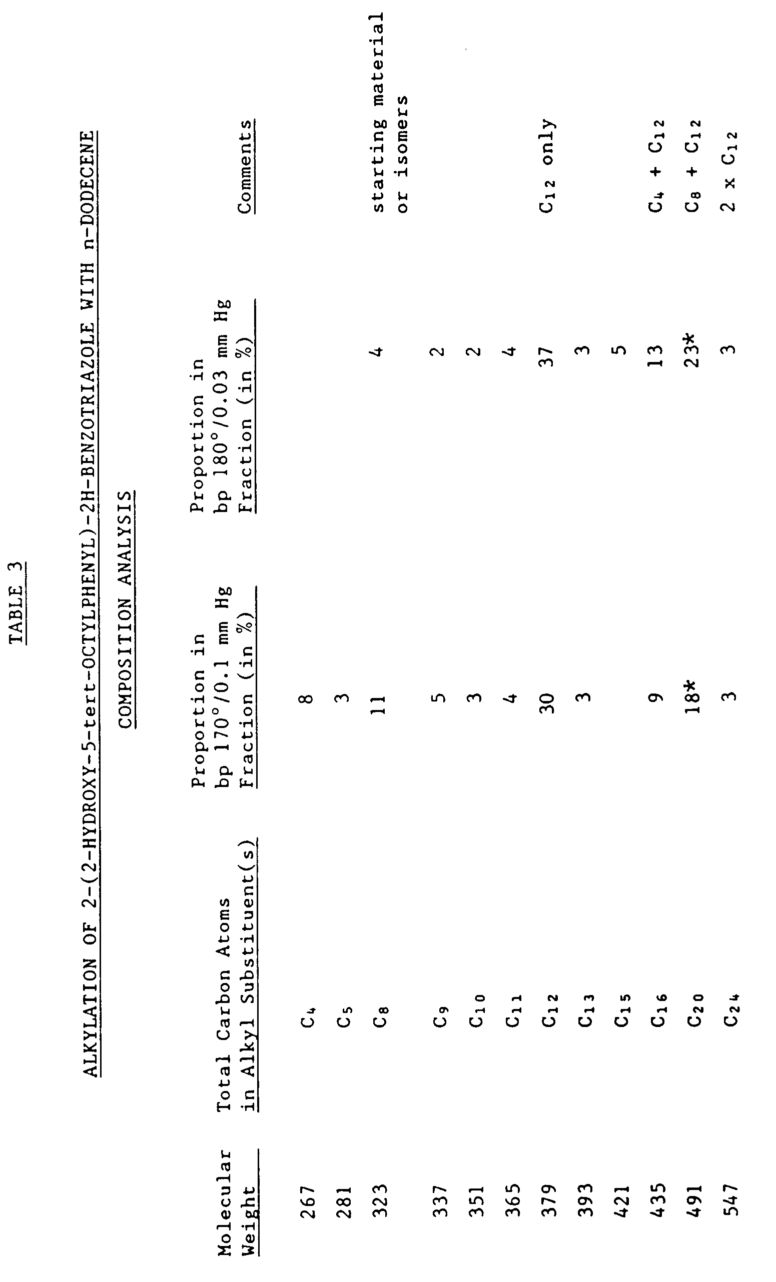

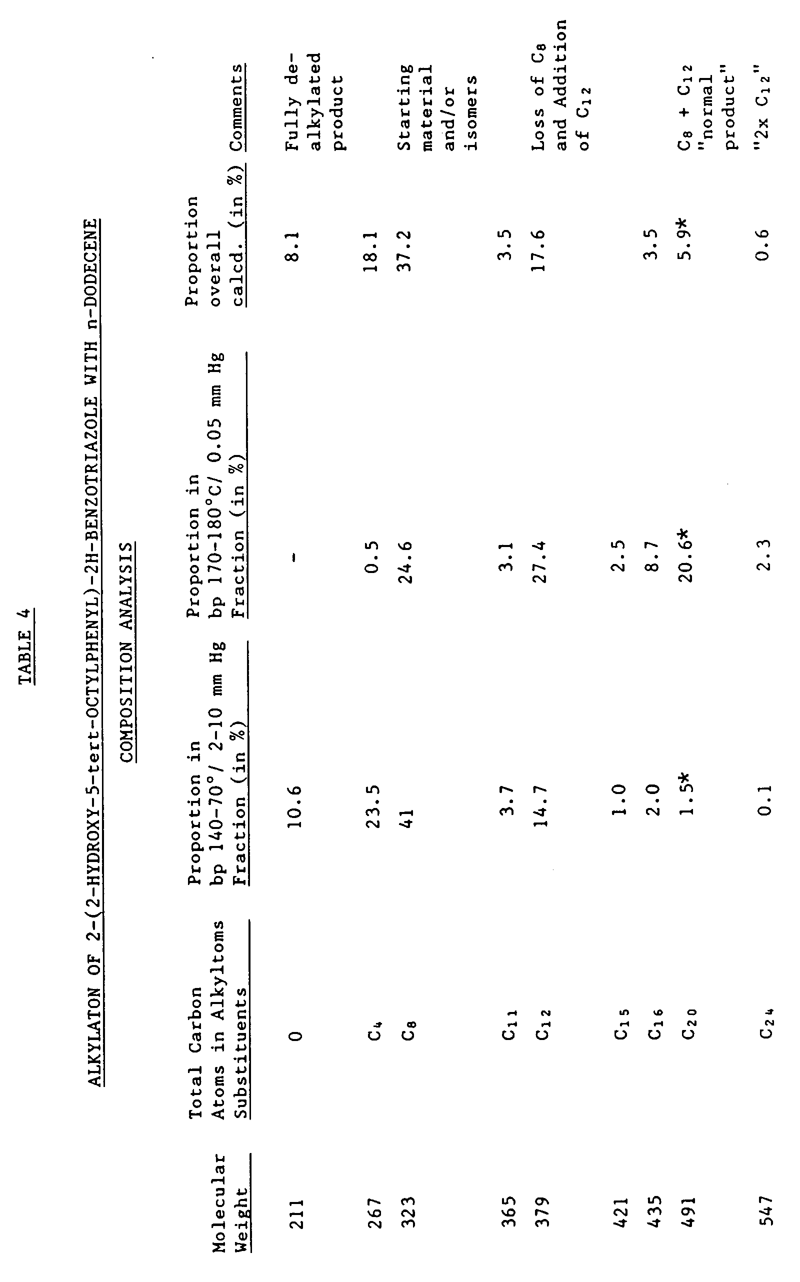

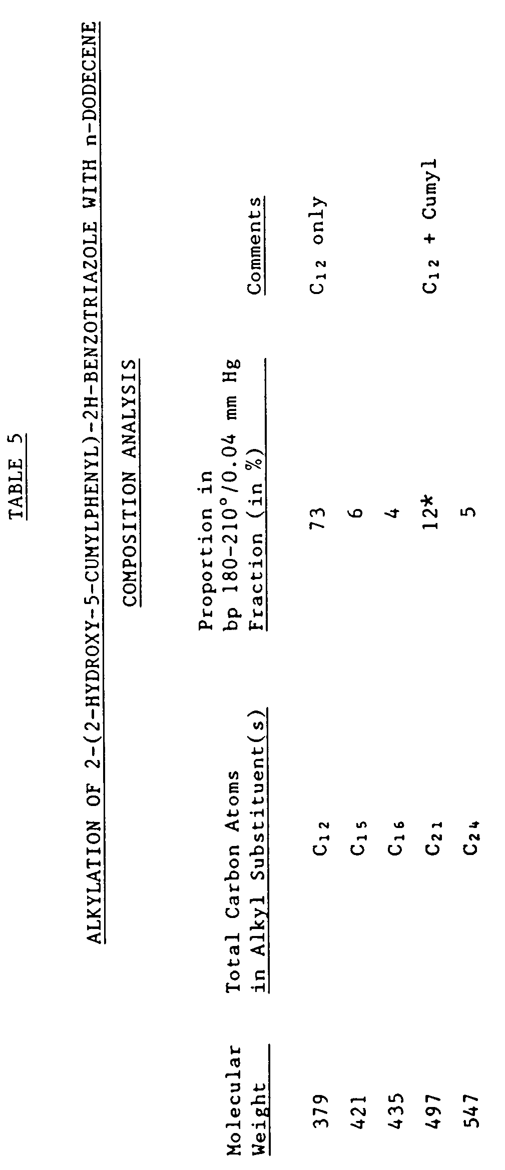

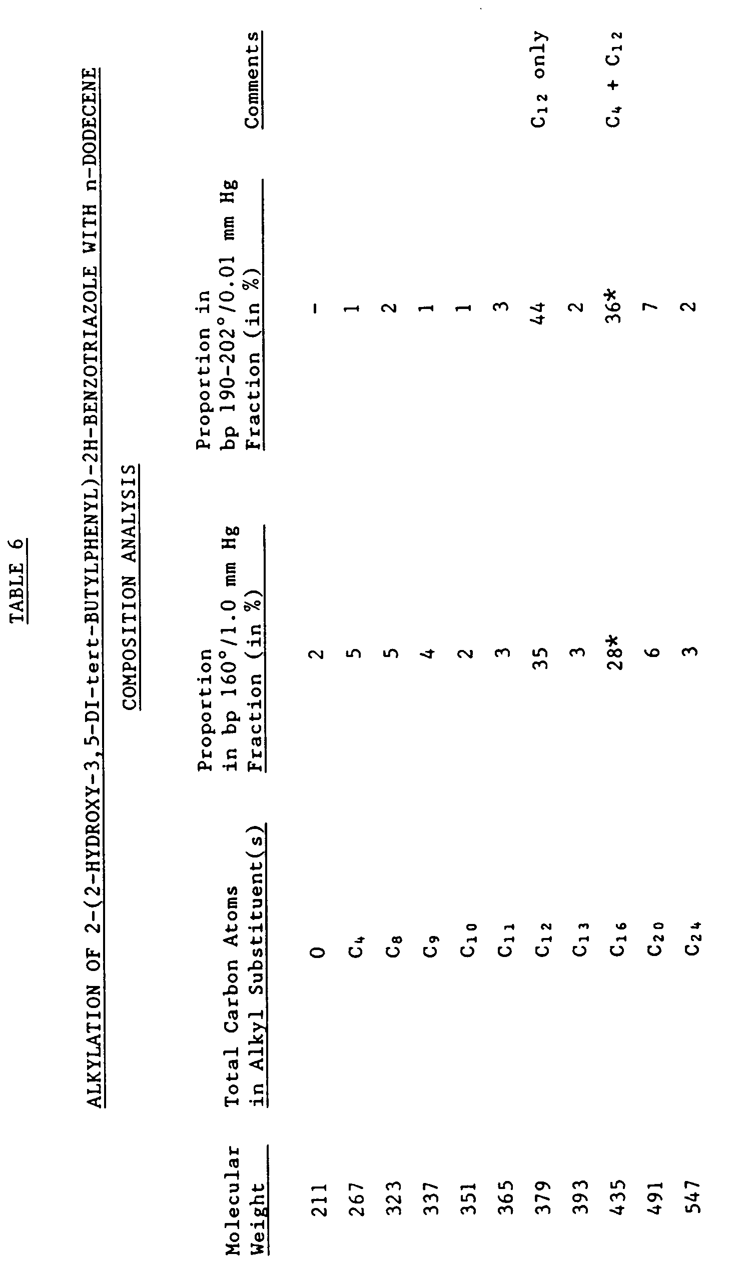

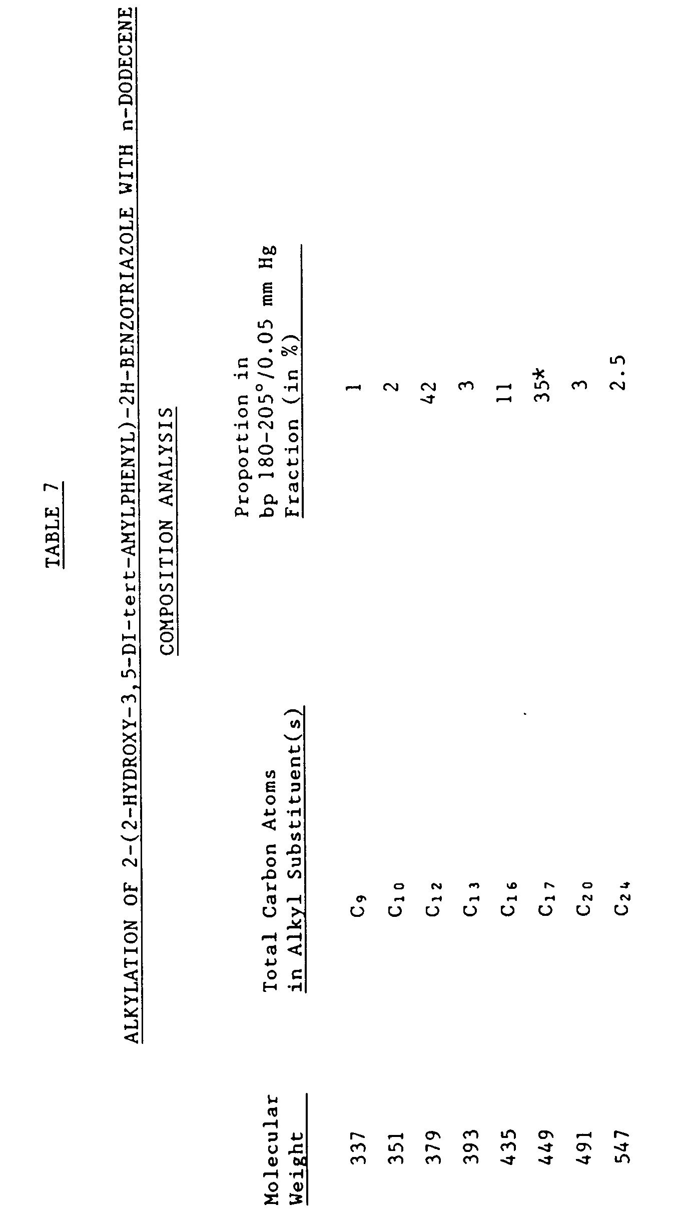

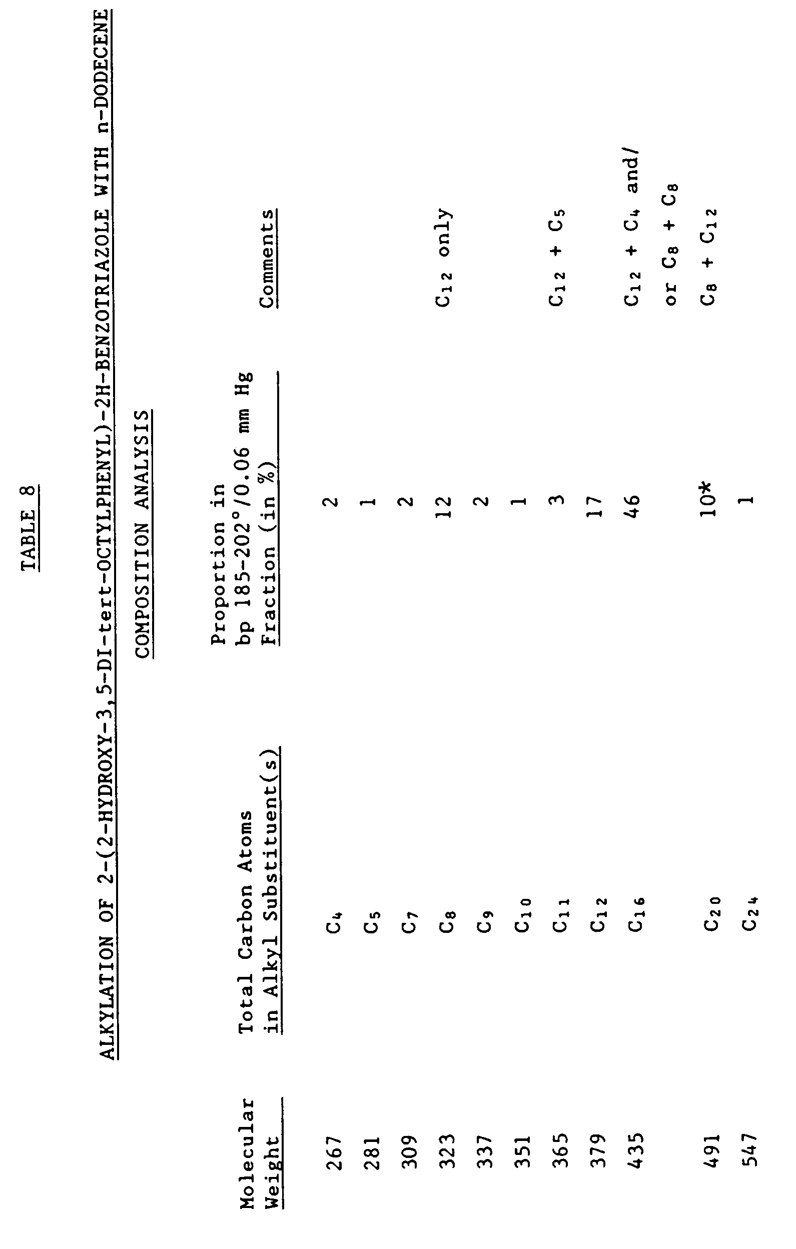

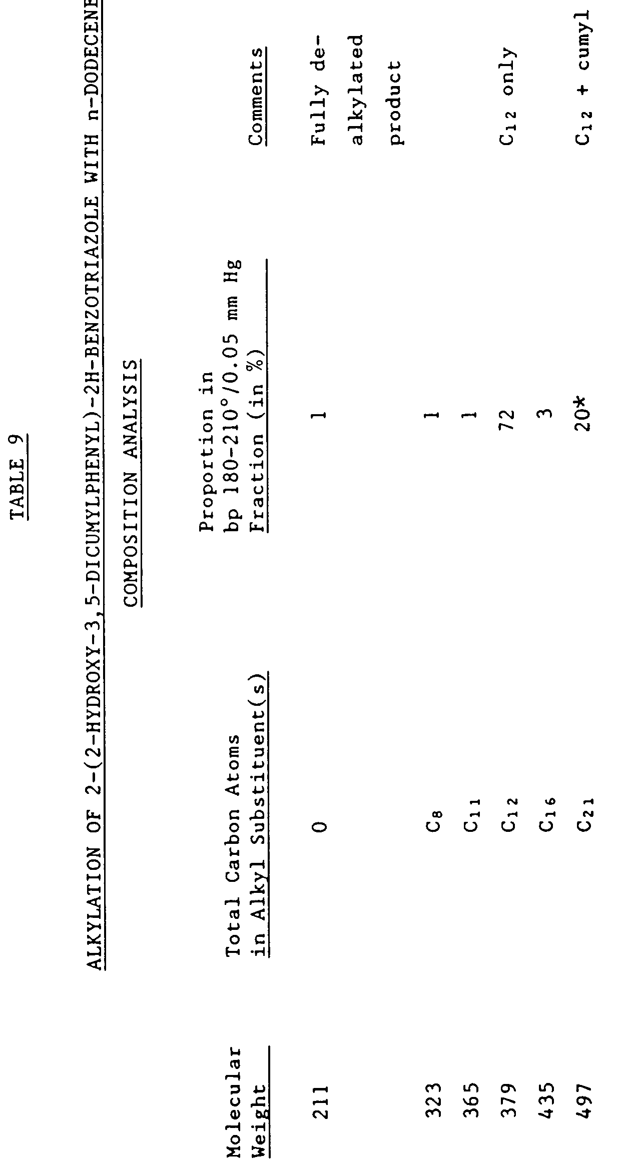

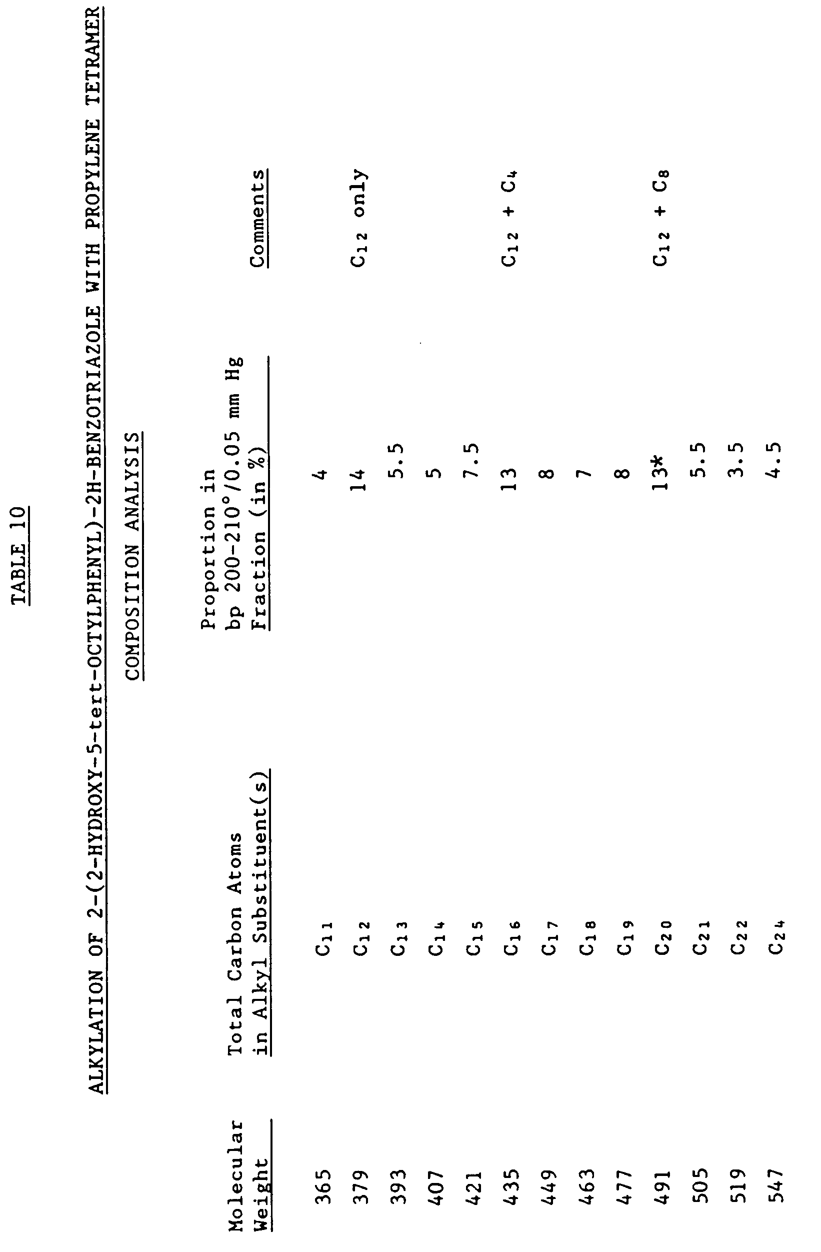

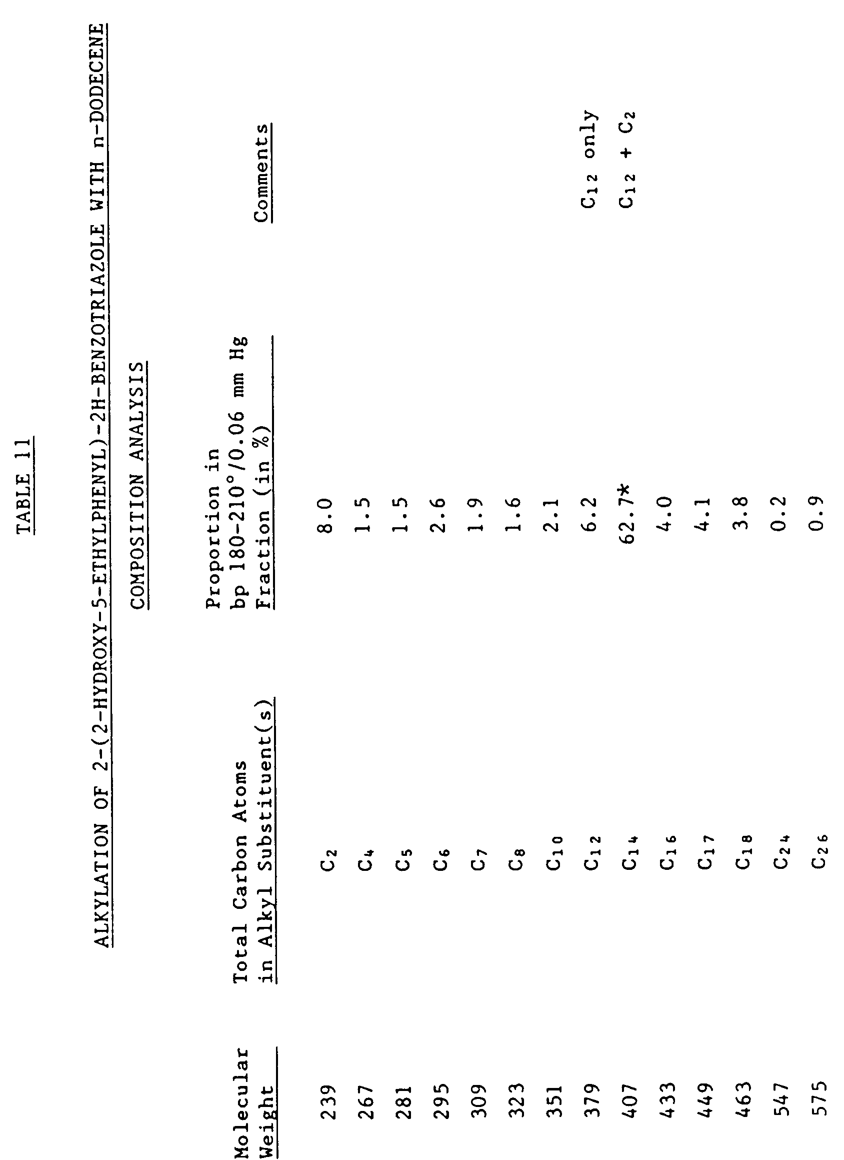

Example 12: To a 1-liter reaction flask fitted with a nitrogen blanket, stirrer, dosing device with proportion pump and distillation column connected with a condensation trap and water jet vacuum pump is charged 323 grams (1 mol) of 2-(2-hydroxy-3,5-di-tert-butylphenyl)-2H-benzotriazole and 96.1 grams (1 mol) of methanesulfonic acid. The reaction flask is swept with nitrogen. Then, without stirring, the mixture is melted by heating to about 150°C. Heating is now continued with stirring to a temperature of 175° C. At this temperature, 234 grams (1.02 mol) of n-hexadecene are added below the surface of the mixture through the dosing device over a 6-hour period. The reaction mixture is then heated for another 30 minutes at 175° C. The mixture is then cooled to about 95 °C and extracted with 100 ml of water, 100 ml of 4 % sodium bicarbonate solution and again with 100 ml of water. Then, 100 grams of bleaching earth (Prolith Rapid) are added and the water is distilled off at 100° C/20 mbar. The bleaching earth is removed by filtration to give 532 grams (corresponding to a yield of 95 % of theory) of a yellow to brownish-yellow liquid. This liquid is further purified by distillation in a thin-film evaporator at 285° C/1-3 mbar. The yellow liquid product has an _{nD}²⁰ of 1.5362.

Examples 13 - 17: Using the general procedure of Example 12, alkylation of 2-(2-hydroxy-3,5-di-tert-butylphenyl)-2H-benzotriazole with other 1-alkenes affords the following liquid products.

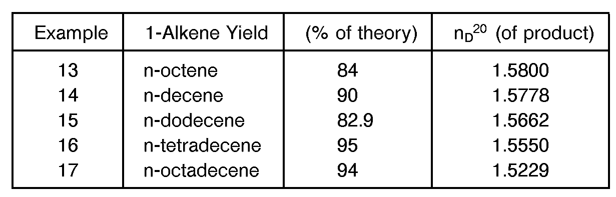

Example 18: Following the general procedure of Example 12, 286.3 grams (0.8 mol) of 5-chloro-2-(2-hydroxy-3,5-di-tert-butylphenyl)-2H-benzotriazole are reacted with 165 grams (0.84 mol) of n-tetradecene in the presence of 96.1 grams (1 mol) of methanesulfonic acid to give a liquid product in a yield of 86 % of theory and with an n8° of 1.5450.

Example 19: Following the procedure of Example 12, 5-chloro-2-(2-hydroxy-3,5-di-tert-butylphenyl)-2H-benzotriazole is reacted with n-octadecene to give a liquid product with an no of 1.5378.

Example 20: Following the general procedure of Example 12, 323 grams (1 mol) of 2-(2-hydroxy-5-tert-octylphenyl)-2H-benzotriazole are alkylated with 171.5 grams (1.02 mol) of n-dodecene in the presence of 96.1 grams (1 mol) of methanesulfonic acid to give a liquid product in a 92 % yield of theory with an nₒ²⁰ of 1.5678.

Examples 21 - 25: In like manner as in Example 20, 2-(2-hydroxy-5-tert-octylphenyl)-2H-benzotriazole is alkylated with other 1-alkenes to give the following products.

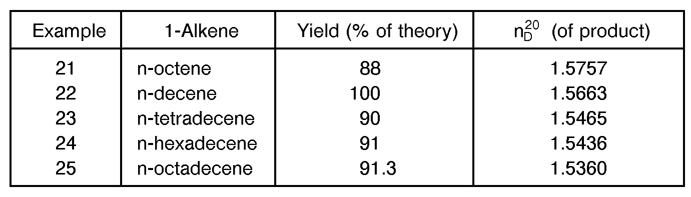

Example 26: Following the general procedure of Example 12, 351 grams (1 mol) of 2-(2-hydroxy-3,5-di-tert-amylphenyl)-2H-benzotriazole are reacted with 200.3 grams (1.02 mol) of n-tetradecene in the presence of 96.1 grams (1 mol) of methanesulfonic acid to give a liquid product in a yield of 88 % of theory with an n_{D}²⁰ of 1.5293.

Examples 27 - 30: In the same manner as in Example 26, 2-(2-hydroxy-3,5-di-tert-amylphenyl)-2H-benzotriazole is alkylated with other 1-alkenes to give the following liquid products.

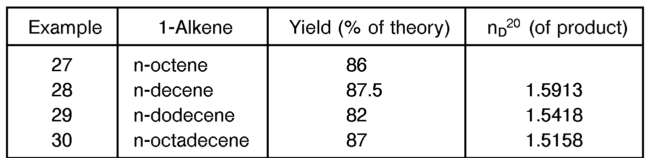

Example 31: Following the procedure of Example 12, 315.8 grams (1 mol) of 5-chloro-2-(2-hydroxy-3-tert-butyl-5-methyl-phenyl)-2H-benzotriazole are alkylated with 176.5 grams (1.05 mol) of n-dodecene in the presence of 96.1 grams (1 mol) of methanesulfonic acid to give a liquid product in a yield of 57 % of theory with an n_{D}²⁰ of 1.5852.

Example 32: To a 1.5 liter reaction flask fitted with a nitrogen blanket, stirrer, dosing device with proportion pump and distillation column connected with a water jet vacuum pump is charged 253.3 grams (1 mol) of 2-(2-hydroxy-5-isopropylphenyl)-2H-benzotriazole and 96.1 grams (1 mol) of methanesulfonic acid. The reaction flask is swept with nitrogen. Then, while slowly stirring, the mixture is heated to 175°C. At this temperature, 420.8 grams (2.5 moles) of n-dodecene are added, while vigoroulsy stirring below the surface of the mixture through the dosing device over a 6 hour-period. The reaction mixture is stirred for another 30 minutes at 175° C. Then, the concentration of the 2-(2-hydroxy-5-isopropylphenyl)-2H-benzotriazole starting material is determined by thin layer chromatography (TLC). The content of the starting material should not exceed 5 %. If the content is higher, further dodecene can be added.

After cooling the batch to about 90°C, 96 ml of water are slowly added and stirred for 10 minutes. Then, the methanesulfonic acid phase (lower phase) is separated. The upper phase containing the crude end product is neutralized with a 4 % sodium bicarbonate solution. After separating the aqueous phase the organic phase is extracted with 100 ml of water. Then, 10 grams of bleaching earth (Prolith Rapid) are added and water is distilled off at 120°C/5 mbar. The bleaching earth is removed by filtration. From the clear product liquid oligomers are removed by distillation with a 0.02 m² thin-film evaporator at 240° C/1-3 mbar. The remaining product is further purified by shorth-path distillation at 240°C/1.10⁻² mbar affording 313 grams (yield: 75 % of theory) of the desired mixture. The yellow liquid product has an n8° of 1.5677.

The distillate of the thin layer distillation is crystallized at 0°C for recovering 19 grams of 2-(2-hydroxy-5-isopropylphenyl)-2H-benzotriazole starting material, corresponding to 8 %, based on the initial amount.

From the methanesulfonic acid phase further starting material is recovered by extraction with 100 ml of n-hexane and subsequent removing the hexane by distillation, affording a further 40 grams of 2-(2-hydroxy-5-isopropylphenyl)-2H-benzotriazole. The extracted methanesulfonic acid solution (ca. 185 grams) is placed into a distillation device for vacuum distillation. At 100 mbar, water (ca. 85 ml) is removed at a head temperature of 55 °C. Then, at a vacuum of 2 mbar about 7.5 grams of aqueous methanesulfonic acid (10-20 %) are distilled off which are added to the next distillation. Subsequently, pure methanesulfonic acid is distilled at a head temperature of 125-130 °C and 2 mbar, affording 87 grams of pure methanesulfonic acid which can be added to a further alkylation batch.

Example 33: Following the general procedure of Example 32, 239.3 grams (1 mol) of 2-(2-hydroxy-5-ethylphenyl)-2H-benzotriazole are reacted with 420.8 grams (2.5 mol) of n-dodecene to give 295 grams (73 % of theory) of a pale yellow liquid product with an n8° of 1.5700.

Example 34: Following the general procedure of Example 32, 267.3 grams (1 mol) of 2-(2-hydroxy-5- sec.-butylphenyl)-2H-benzotriazole are reacted with 420.8 grams (2.5 mol) of n-dodecene to give 232 grams (53 % of theory) of a yellow liquid product with an n8° of 1.5612.

Example 35: Following the general procedure of Example 32, 247.7 grams (1 mol) of 2-(2-hydroxy-5-chlorophenyl)-2H-benzotriazole are reacted with 505 grams of n-dodecene to give 186 grams (45 % of theory) of an orange liquid product with an n8° of 1.5632.

Example 36: Following the general procedure of Example 32, 301.4 grams (1 mol) of 2-(2-hydroxy-5-benzylphenyl)-2H-benzotriazole is reacted with 420.8 grams (2.5 mol) of n-dodecene to give 292 grams (63 % of theory) of an orange liquid product with an n8° of 1.5762.

Example 37: Following the general procedure of Example 32, 253.3 grams (1 mol) of 2-(2-hydroxy-5-isopropyl)-2H-benzotriazole is reacted with 350.6 grams (2.5 mol) of n-decene to give the liquid alkylated product in a yield of 75 % of theory with an n8° of 1.5700.

Example 38: Example 37 is repeated, however replacing the n-decene by 491 grams (2.5 mol) of n-tetradecene. The resulting liquid alkylated product is obtained in a yield of 70 % of theory and with an n8° of 1.5560.

Example 39: Following the general procedure of Example 32, 239.3 grams (1 mol) of 2-(2-hydroxy-5-ethylphenyl)-2H-benzotriazole are reacted with 350.6 grams (2.5 mol) of n-decene to give the liquid alkylated product in a yield of 85 % of theory with an n8° of 1.5771.

Example 40: Example 39 is repeated, however replacing the n-decene by 491 grams (2.5 mol) of n-tetradecene. The resulting liquid alkylated product is obtained in a yield of 85 % of theory and with an n8° of 1.5358.

Example 41: Following the general procedure of Example 32, 267.3 grams (1 mol) of 2-(2-hydroxy-sec.- butylphenyl)-2H-benzotriazole are reacted with 350.6 grams (2.5 mol) of n-decene to give the liquid alkylated product in a yield of 47 % of theory with an n8° of 1.5663.

Example 42: Example 41 is repeated, however replacing the n-decene by 491 grams (2.5 mol) of n-tetradecene. The resulting liquid alkylated product is obtained in a yield of 60 % of theory and with an n8° of 1.5554.

Example 43: A thermoset acrylic resin coating composition, typical for automotive topcoats, is formulated with 2 % by weight of the light stabilizer prepared in Example 2. The coating composition is applied to a metal panel and baked at 130°C to cure the resin. The coated panel is then exposed to accelerated (quick) weathering test (QUV) involving alternating 8-hour period of UV irradiation at 70 °C with a 4-hour period of condensation (rain) at 50 _{°} C for each cycle.

The 20 ° gloss (ASTM D523 and D2457) and the Distinctness of Image (ASTM E 430) values for the coating before and after weathering in the QUV test are measured and the % retention of 20 ° gloss and of distinctness of image (D/I) are calculated. The control is the same thermoset acrylic resin coating containing no stabilizer.

The stabilized coating exhibits far superior gloss and D/I retention than the unstabilized control. The stabilized sample shows no sign of surface cracking or crazing again showing the efficacy of the instant compounds as light stabilizers.

Example 44: An oil-modified urethane varnish containing 2 % by weight of the stabilizer prepared in Example 7 is coated on an aluminum panel and exposed outdoors at a 90 ° angle facing south in Southern New York for a period of 10.5 months.

The yellowness index (YI), measured by ASTM D 1925, for the sample is measured before exposure and after exposure. The change in YI is a measure of how much the urethane coating discolored over the test period. The lower the change in YI the less discolored is the sample.

The urethane varnish containing the instant benzotriazoles of Example 7 does not yellow whereas the control (without stabilizer) turns perceptily discolored (yellowed).

Example 45: Haze Development in Photographic Compositions
The direct assessment of the compatibility of benzotriazole light stabilizers in photographic composition is difficult. The compositions containing such stabilizers in photographic oils often take extended periods of time for separation or haze to be observed.

An important property of photographic compositions directly related to such compatibility parameters is haze. For the preparation of clear and precise photographic images, haze must obviously be minimized or better yet essentially eliminated.

Using the procedure described in U.S. Patent No. 4,383,863, Example 5, a UV-protecting layer is prepared in gelatin containing an anionic wetting agent, a hardener and the instant stabilizer of Example 10 using no solvent.

A very fine dispersion of the instant stabilizer in this gelatin composition is produced by ultrasonic mixing to give a UV-protecting layer which is clear and transparent and exhibits no haze.

Example 46: Some of the liquid benzotriazole mixtures of this invention are processed in a curable coating composition. The respective mixtures are, as a solution in xylene, incorporated into an ac- rylate/melamine clear coat in such a way that their content corresponds to 2 %, relative to the solids content of the clear coat.

The clear coat used has the following composition:

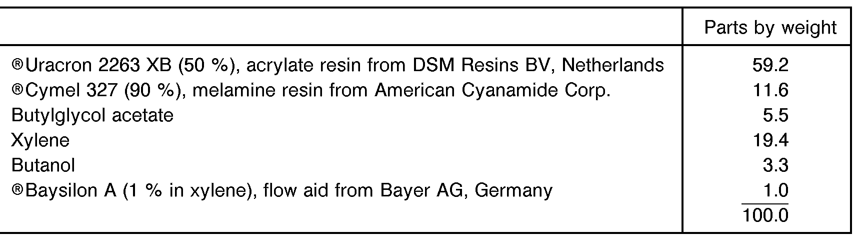

The coating composition obtained (containing 2 % by weight, based on the solids content, of the respective benzotriazole mixture) is diluted with a 13:6:1 mixture of xylene/butanol/butylglycol acetate up to sprayability, sprayed onto a prepared aluminium sheet (coil coat, filler, silver metallic base coat, based on polyester/cellulose-acetobutyrate/melamine binder) and baked at 130°C for 30 minutes. This gives a dry film thickness of 40-45 µm of clear coat.

The test specimens are subjected to an accelerated weathering treatment in an @Uvcon weathering apparatus from Atlas Corp. using the following weathering cycle: 4 hours UV-irradiation at 60 °C, 4 hours condensation (rain) at 50 °C. The 20 gloss retention (in %) is measured according to DIN 67530. The results are compiled in Table 12

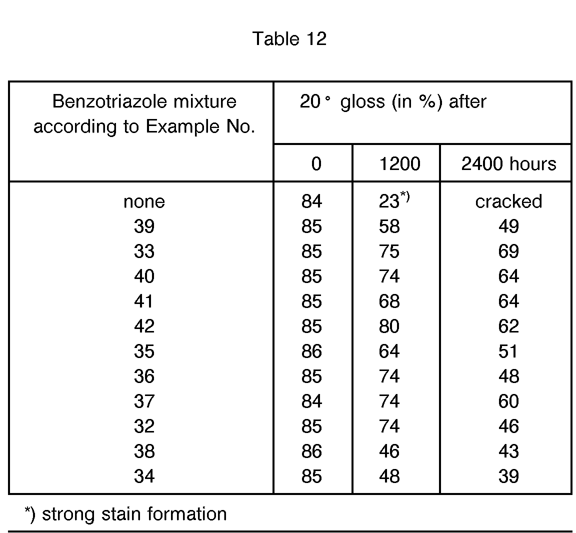

Example 47: Some of the liquid benzotriazole mixtures of this invention are processed in a curable coating composition. The respective mixtures are, as a solution in xylene, incorporated into an ac- rylate/melamine clear coat in such a way that their content corresponds to 2 %, relative to the solids content of the clear coat.

The clear coat used has the following composition:

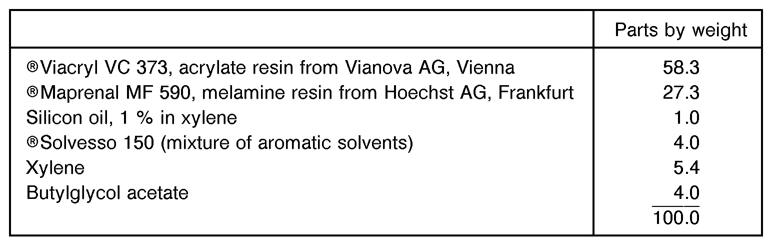

The coating composition obtained (containing 2 % by weight, based on the solids content, of the respective benzotriazole mixture) is diluted with a 13:6:1 mixture of xylene/butanol/butylglycol acetate up to sprayability, sprayed onto a prepared aluminium sheet (coil coat, filler, silver metallic base coat, based on polyester/cellulose-acetobutyrate/melamine binder) and baked at 130°C for 30 minutes. This gives a dry film thickness of 40-45 µm of clear coat.

The test specimens are then subjected to an accelerated weathering treatment in an Xenotest 1200 weathering apparatus from Heraeus, Hanau, FRG, using the following weathering cycle: 10 minutes dry UV-irradiation at 70±2 _{°} C; 5 minutes UV-irradiation with condensation (rain) at 70±2 _{°} C. The 20 ° gloss retention (in %) is measured according to DIN 67530. The results are listed in Table 13

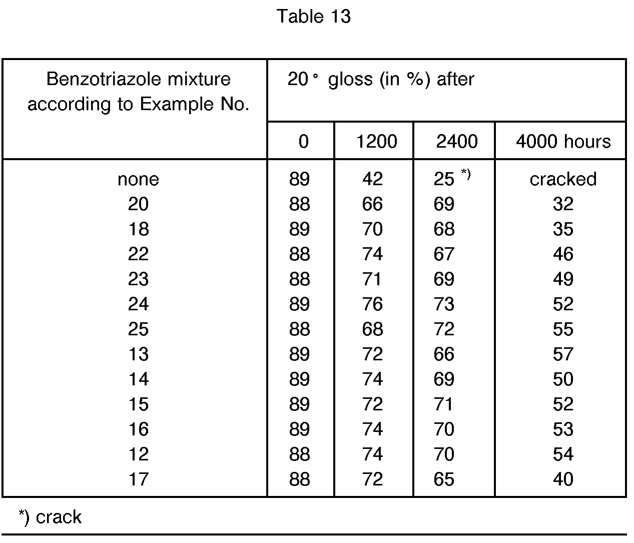

Example 48: Stabilization of a magenta layer
a) A gelatin layer containing silver bromide and a magenta coupler is first applied to a paper base material coated with polyethylene (Ag Br layer). A further gelatin layer containing a UV absorber mixture according to the invention is applied onto a transparent polyester sheet (covering layer). The magenta coupler containing layer is covered by the sheet containing the UV absorber mixture.

The gelatin layers consist of the following components (per m² of base material or sheet respectively):

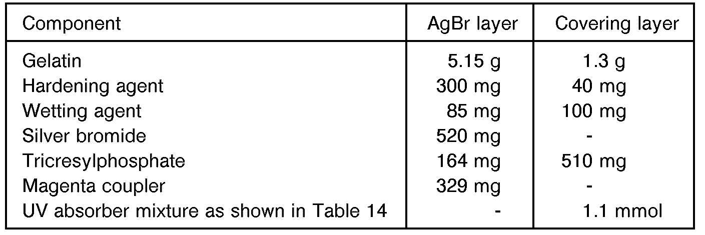

The magenta coupler has the following structure: 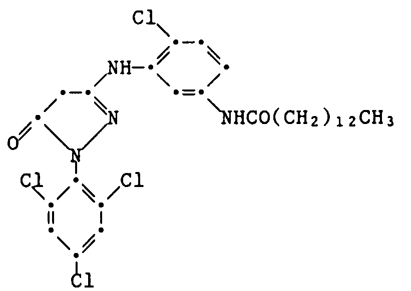 2,4-Dichloro-6-hydroxytriazine is used as the hardening agent. The sodium salt of diisobutylnaph- thalenesulfonic acid is used as the wetting agent.
b) A step wedge having a density difference of 0.15 loge per step is exposed to light on top of each of the sample obtained according to a) above, and the procedure specified in the instructions of the manufacturer in the Kodak process EP2 for negative colour paper is then followed.

To determine the yellowing, the diffuse reflectance density in the blue is then measured. The wedge is then exposed to a total of 15 kJ/cm² in an Atlas exposure instrument, the diffuse reflectance density (in the blue) is measured again and the increase in yellow dye is calculated 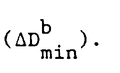 The results with various UV absorber mixtures are shown in Table 14.

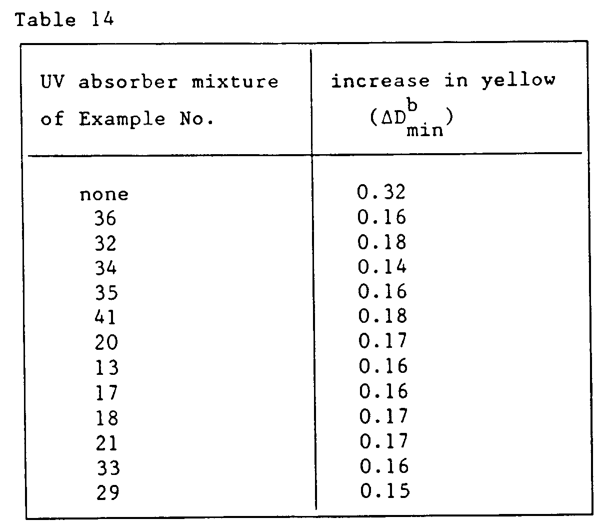

Example 49: Stabilization of a yellow layer
The procedure of Example 48 is repeated, but using, instead of the magenta coupler, the yellow coupler A of the following formula: 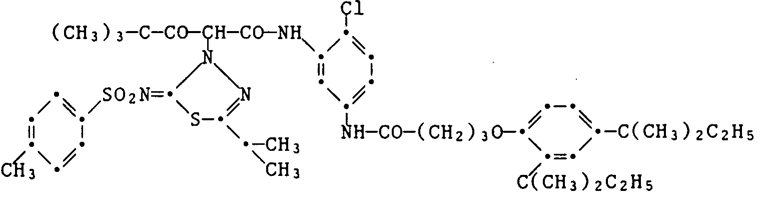 The gelatin layers have the following composition (per m²):

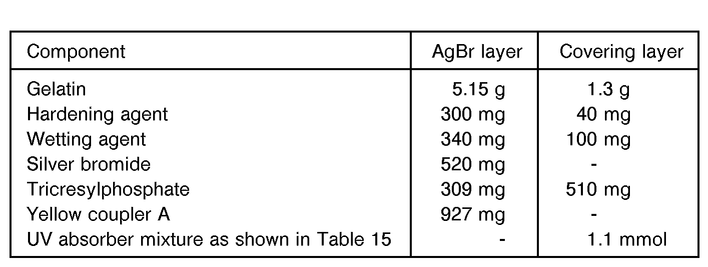

After Exposure and processing as described in Example 48, the diffuse reflectance density in the blue is measured for the yellow step at a density of the wedge between 0.9 and 1.1. The wedge is then exposed at a total of 15 kJ/cm² in an Atlas exposure instrument and the diffuse reflectance density is determined again.

The percentage losses in optical density listed in Table 15 can be calculated on the basis of the values thus obtained.

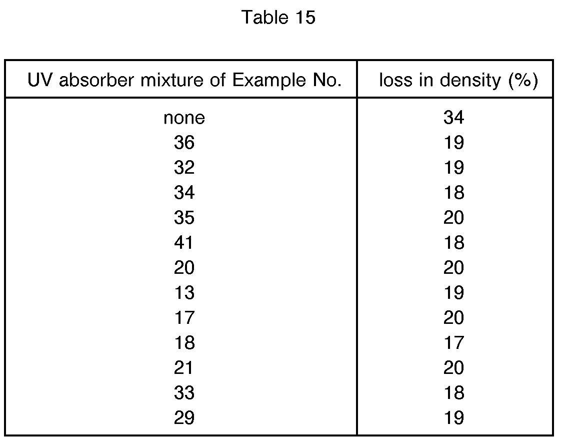

Example 50: Stabilization of a yellow layer
The procedure of Example 48 is repeated, but using, instead of the magenta coupler, the yellow coupler B of the following formula: 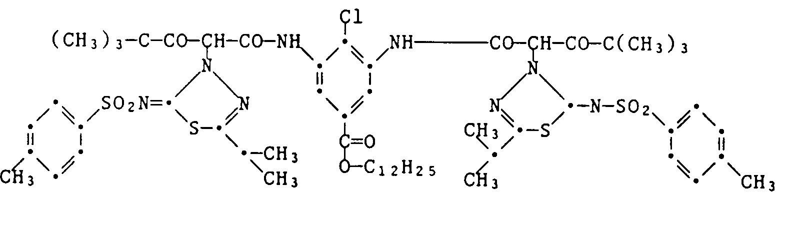 The gelatin layers have the following composition (per m²):

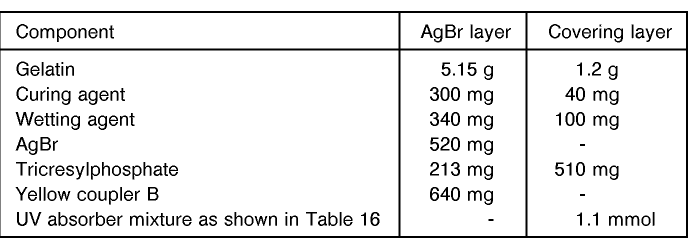

After Exposure and processing as described in Example 48, the diffuse reflectance density in the blue is measured for the yellow step at a density of the wedge between 0.9 and 1.1. The wedge is then exposed at a total of 15 kJ/cm² in an Atlas exposure instrument and the diffuse reflectance density is determined again.

The percentage losses in density listed in Table 16 can be calculated on the basis of the values thus obtained.

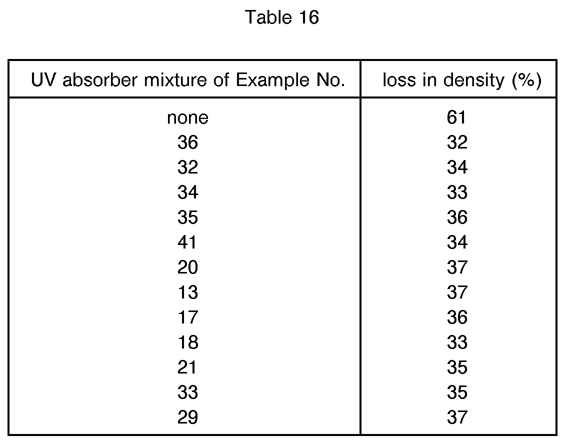

Example 51: Stabilization of a cyan layer
The procedure of Example 48 is repeated, but using, instead of the magenta coupler, the cyan coupler of the following formula: 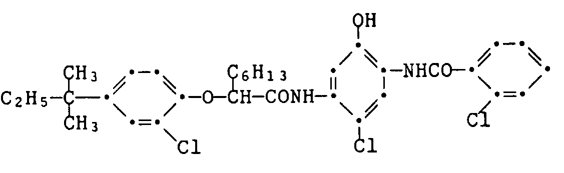 The gelatin layers have the following composition (per m²):

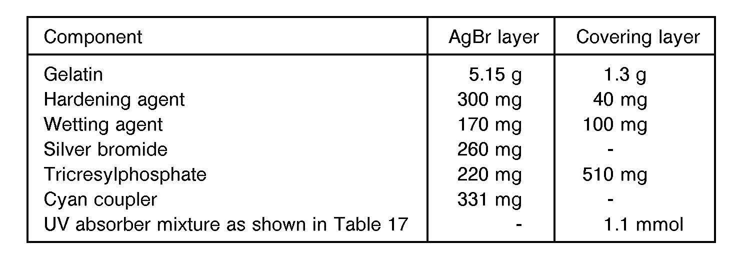

After Exposure and processing as described in Example 48, the diffuse reflectance density in the red is determined for the cyan step at a density of the wedge between 0.9 and 1.1. The wedge is then exposed at a total of 15 kJ/cm² in an Atlas exposure instrument and the diffuse reflectance density is determined again.

The percentage losses in density listed in Table 17 can be calculated on the basis of the values thus obtained.

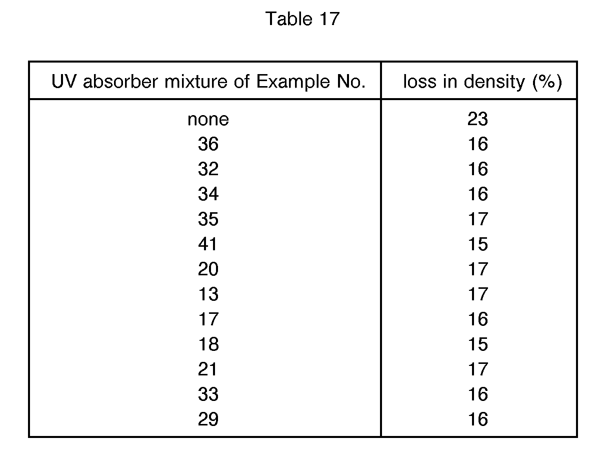

## Claims (Claims for the following Contracting State(s) : BE, CH, DE, FR, GB, IT, LI, NL)

1. A process for preparing a normally liquid or non-crystalline mixture of 2-hydroxyphenyl-2H-benzotriazoles which process comprises reacting a 2H-benzotriazole of the formula (A) or (B) 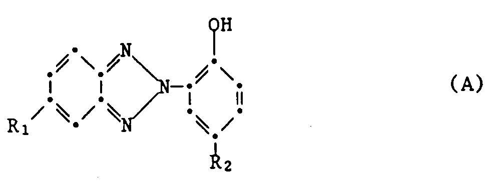 or 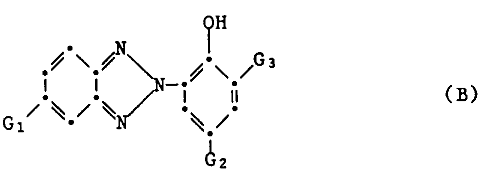 wherein
R₁ is hydrogen, chloro, alkyl of 1 to 4 carbon atoms or alkoxy of 1 to 4 carbon atoms,
R₂ is alkyl of 2 to 12 carbon atoms, cycloalkyl of 5 to 8 carbon atoms, aralkyl of 7 to 9 carbon atoms or chlorine,
G₁ has the same meaning as Ri, and
G₂ and G₃ are independently branched alkyl of 3 to 12 carbon atoms, cycloalkyl of 5 to 8 carbon atoms or aralkyl of 7 to 9 carbon atoms, or one of G₂ and G₃ is methyl, ethyl or chlorine when the other of G₂ and G₃ is branched alkyl, cycloalkyl or aralkyl as defined above,
with a straight or branched chain alkene of 8 to 40 carbon atoms or mixture of said alkenes in the presence of an acidic catalyst at a temperature of 100 to 200 _{°} C.

2. A process according to claim 1 wherein R₂ or G₂ and G₃ are independently branched alkyl of 3 to 12 carbon atoms or aralkyl of 7 to 9 carbon atoms, or R₂ is ethyl or chlorine, or one of G₂ or G₃ is methyl, ethyl or chlorine and the other of G₂ or G₃ is branched alkyl of 3 to 12 carbon atoms.

3. A process according to claim 1 wherein the temperature is 140 - 180 _{°} C.

4. A process according to claim 1 wherein the alkene is of 8 to 16 carbon atoms.

5. A process according to claim 1 wherein the equivalent ratio of alkene to 2H-benzotriazole is 1:1 to 6:1.

6. A process according to claim 1 wherein the acidic catalyst is selected from the group consisting of aliphatic, aromatic and substituted aromatic sulfonic acids, sulfuric acid, phosphoric acid, acidic clays and acidic molecular sieves.

7. A process according to claim 6 wherein the acidic catalyst is an aliphatic sulfonic acid.

8. A process according to claim 1 wherein the equivalents of acidic catalyst to equivalents of benzotriazole is 0.2 to 3.

9. A normally liquid or non-crystalline mixture of benzotriazoles obtainable according to the process of claim 1.

10. A stabilized composition which comprises
(a) an organic material, and
(b) a mixture as defined in claim 9, as stabilizer.

11. A stabilized composition according to claim 10 wherein the organic material (a) is an organic polymer.

12. A stabilized composition according to claim 11 wherein the organic polymer is a polyolefin, a styrene polymer, a polyacrylate, a polyamide, a polyurethane, a halogen containing vinyl polymer, an alkyd resin, a thermoset acrylic resin or an epoxy resin.

13. A stabilized composition according to claim 12 wherein the organic material is in the form of photographic material or part of a photographic element.

14. Use of a mixture as defined in claim 9 as stabilizer for organic material.

## Claims (Claims for the following Contracting State(s) : ES)

1. A process for preparing a normally liquid or non-crystalline mixture of 2-hydroxyphenyl-2H-benzotriazoles which process comprises reacting a 2H-benzotriazole of the formula (A) or (B) 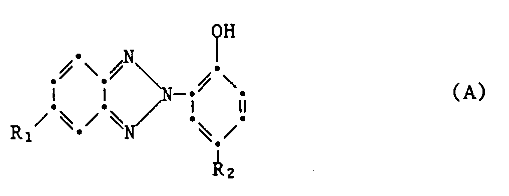 or 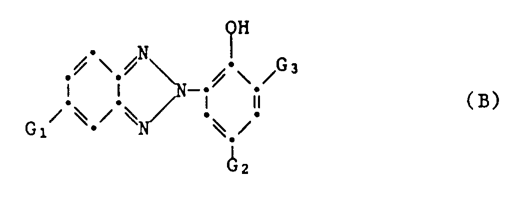 wherein
R₁ is hydrogen, chloro, alkyl of 1 to 4 carbon atoms or alkoxy of 1 to 4 carbon atoms,
R₂ is alkyl of 2 to 12 carbon atoms, cycloalkyl of 5 to 8 carbon atoms,
aralkyl of 7 to 9 carbon atoms or chlorine,
G₁ has the same meaning as Ri, and
G₂ and G₃ are independently branched alkyl of 3 to 12 carbon atoms, cycloalkyl of 5 to 8 carbon atoms or aralkyl of 7 to 9 carbon atoms, or one of G₂ and G₃ is methyl, ethyl or chlorine when the other of G₂ and G₃ is branched alkyl, cycloalkyl or aralkyl as defined above,
with a straight or branched chain alkene of 8 to 40 carbon atoms or mixture of said alkenes in the presence of an acidic catalyst at a temperature of 100 to 200 _{°} C.

2. A process according to claim 1 wherein R₂ or G₂ and G₃ are independently branched alkyl of 3 to 12 carbon atoms or aralkyl of 7 to 9 carbon atoms, or R₂ is ethyl or chlorine, or one of G₂ or G₃ is methyl, ethyl or chlorine and the other of G₂ or G₃ is branched alkyl of 3 to 12 carbon atoms.

3. A process according to claim 1 wherein the temperature is 140 - 180° C.

4. A process according to claim 1 wherein the alkene is of 8 to 16 carbon atoms.

5. A process according to claim 1 wherein the equivalent ratio of alkene to 2H-benzotriazole is 1:1 to 6:1.

6. A process according to claim 1 wherein the acidic catalyst is selected from the group consisting of aliphatic, aromatic and substituted aromatic sulfonic acids, sulfuric acid, phosphoric acid, acidic clays and acidic molecular sieves.

7. A process according to claim 6 wherein the acidic catalyst is an aliphatic sulfonic acid.

8. A process according to claim 1 wherein the equivalents of acidic catalyst to equivalents of benzotriazole is 0.2 to 3.

9. A stabilized composition which comprises
(a) an organic material, and
(b) a mixture obtainable according to claim 1, as stabilizer.

10. A stabilized composition according to claim 9 wherein the organic material (a) is an organic polymer.

11. A stabilized composition according to claim 10 wherein the organic polymer is a polyolefin, a styrene polymer, a polyacrylate, a polyamide, a polyurethane, a halogen containing vinyl polymer, an alkyd resin, a thermoset acrylic resin or an epoxy resin.

12. A stabilized composition according to claim 11 wherein the organic material is in the form of photographic material or part of a photographic element.

## Patentansprüche (Patentansprüche für folgende(n) Vertragsstaat(en) : BE, CH, DE, FR, GB, IT, LI, NL)

1. Verfahren zur Herstellung eines normalerweise flüssigen oder nichtkristallinen Gemisches von 2-Hydroxyphenyl-2H-benzotriazolen, wobei das Verfahren umfaßt Umsetzen eines 2H-Benzotriazols der Formel (A) oder (B) 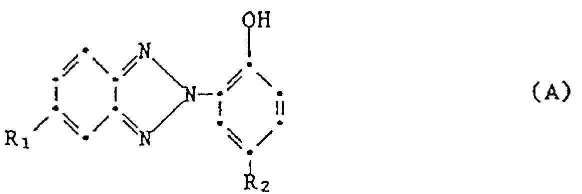 oder 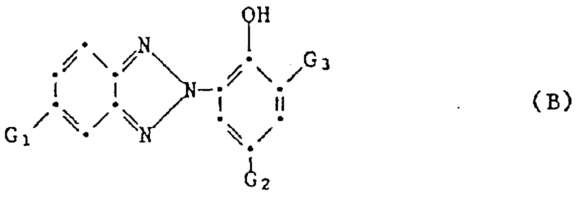 worin R₁ Wasserstoff, Chlor, Alkyl mit 1 bis 4 Kohlenstoffatomen oder Alkoxy mit 1 bis 4 Kohlenstoffatomen bedeutet,
R₂ Alkyl mit 2 bis 12 Kohlenstoffatomen, Cycloalkyl mit 5 bis 8 Kohlenstoffatomen, Aralkyl mit 7 bis 9 Kohlenstoffatomen oder Chlor bedeutet,
G₁ die gleiche Bedeutung hat wie Ri, und
G₂ und G₃, unabhängig voneinander, eine verzweigte Alkylgruppe mit 3 bis 12 Kohlenstoffatomen, Cycloalkyl mit 5 bis 8 Kohlenstoffatomen oder Aralkyl mit 7 bis 9 Kohlenstoffatomen bedeuten oder einer der Reste G₂ und G₃ Methyl, Ethyl oder Chlor bedeutet, wenn der andere Rest von G₂ und G₃, wie vorstehend definiert, eine verzweigte Alkyl-, Cycloalkyl- oder Aralkylgruppe bedeutet,
mit einem gerad- oder verzweigtkettigen Alken mit 8 bis 40 Kohlenstoffatomen oder einem Gemisch der Alkene in Gegenwart eines sauren Katalysators bei einer Temperatur von 100 bis 200 °C.

2. Verfahren nach Anspruch 1, wobei R₂ oder G₂ und G₃, unabhängig voneinander, eine verzweigte Alkylgruppe mit 3 bis 12 Kohlenstoffatomen oder Aralkyl mit 7 bis 9 Kohlenstoffatomen bedeuten, oder R₂ Ethyl oder Chlor darstellt, oder einer der Reste G₂ oder G₃ Methyl, Ethyl oder Chlor bedeutet und der andere Rest G₂ oder G₃ eine verzweigte Alkylgruppe mit 3 bis 12 Kohlenstoffatomen bedeutet.

3. Verfahren nach Anspruch 1, wobei die Temperatur 140-180 _{°} C beträgt.

4. Verfahren nach Anspruch 1, wobei das Alken 8 bis 16 Kohlenstoffatome enthält.

5. Verfahren nach Anspruch 1, wobei das Äquivalentverhältnis von Alken zu 2H-Benzotriazol 1:1 bis 6:1 beträgt.

6. Verfahren nach Anspruch 1, wobei der saure Katalysator ausgewählt ist aus der Gruppe, bestehend aus aliphatischen, aromatischen und substituierten aromatischen Sulfonsäuren, Schwefelsäure, Phosphorsäure, sauren Tonen und sauren Molekularsieben.

7. Verfahren nach Anspruch 6, wobei der saure Katalysator eine aliphatische Sulfonsäure ist.

8. Verfahren nach Anspruch 1, wobei die Äquivalente von saurem Katalysator zu Äquivalenten von Benzotriazol 0,2 bis 3 betragen.

9. Normalerweise flüssiges oder nichtkristallines Gemisch von Benzotriazolen, erhältlich gemäß dem Verfahren von Anspruch 1.

10. Stabilisierte Masse, umfassend
(a) ein organisches Material und
(b) ein Gemisch gemäß Anspruch 9 als Stabilisator.

11. Stabilisierte Masse nach Anspruch 10, wobei das organische Material (a) ein organisches Polymer ist.

12. Stabilisierte Masse nach Anspruch 11, wobei das organische Polymer ein Polyolefin, ein Styrolpolymer, ein Polyacrylat, ein Polyamid, ein Polyurethan, ein Halogen enthaltendes Vinylpolymer, ein Alkydharz, ein duroplastisches Acrylharz oder ein Epoxidharz ist.

13. Stabilisierte Masse nach Anspruch 12, wobei das organische Material in Form eines photographischen Materials oder Teil eines photographischen Elements vorliegt.

14. Verwendung eines Gemisches nach Anspruch 9 als Stabilisator für organisches Material.

## Patentansprüche (Patentansprüche für folgende(n) Vertragsstaat(en) : ES)

1. Verfahren zur Herstellung eines normalerweise flüssigen oder nichtkristallinen Gemisches von 2-Hydroxyphenyl-2H-benzotriazolen, wobei das Verfahren umfaßt
Umsetzen eines 2H-Benzotriazols der Formel (A) oder (B) 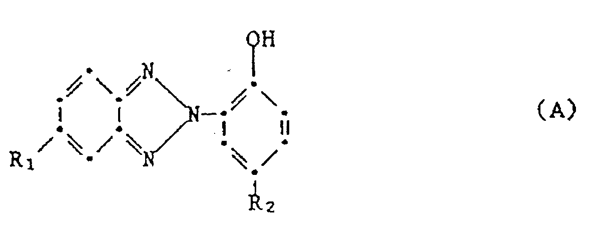 oder 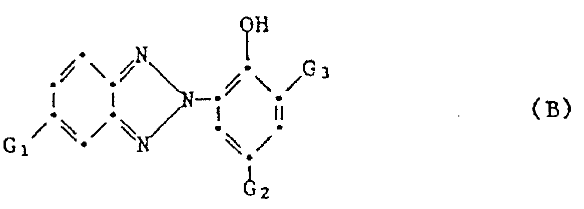 worin R₁ Wasserstoff, Chlor, Alkyl mit 1 bis 4 Kohlenstoffatomen oder Alkoxy mit 1 bis 4 Kohlenstoffatomen bedeutet,
R₂ Alkyl mit 2 bis 12 Kohlenstoffatomen, Cycloalkyl mit 5 bis 8 Kohlenstoffatomen, Aralkyl mit 7 bis 9 Kohlenstoffatomen oder Chlor bedeutet,
G₁ die gleiche Bedeutung hat wie Ri, und
G₂ und G₃, unabhängig voneinander, eine verzweigte Alkylgruppe mit 3 bis 12 Kohlenstoffatomen, Cycloalkyl mit 5 bis 8 Kohlenstoffatomen oder Aralkyl mit 7 bis 9 Kohlenstoffatomen bedeuten oder einer der Reste G₂ und G₃ Methyl, Ethyl oder Chlor bedeutet, wenn der andere Rest von G₂ und G₃, wie vorstehend definiert, eine verzweigte Alkyl-, Cycloalkyl-oder Aralkylgruppe bedeutet,
mit einem gerad- oder verzweigtkettigen Alken mit 8 bis 40 Kohlenstoffatomen oder einem Gemisch der Alkene in Gegenwart eines sauren Katalysators bei einer Temperatur von 100 bis 200 _{°} C.

2. Verfahren nach Anspruch 1, wobei R₂ oder G₂ und G₃, unabhängig voneinander, eine verzweigte Alkylgruppe mit 3 bis 12 Kohlenstoffatomen oder Aralkyl mit 7 bis 9 Kohlenstoffatomen bedeuten, oder R₂ Ethyl oder Chlor darstellt, oder einer der Reste G₂ oder G₃ Methyl, Ethyl oder Chlor bedeutet und dem andere Rest G₂ oder G₃ eine verzweigte Alkylgruppe mit 3 bis 12 Kohlenstoffatomen bedeutet.

3. Verfahren nach Anspruch 1, wobei die Temperatur 140-180 _{°} C beträgt.

4. Verfahren nach Anspruch 1, wobei das Alken 8 bis 16 Kohlenstoffatome enthält.

5. Verfahren nach Anspruch 1, wobei das Äquivalentverhältnis von Alken zu 2H-Benzotriazol 1:1 bis 6:1 beträgt.

6. Verfahren nach Anspruch 1, wobei der saure Katalysator ausgewählt ist aus der Gruppe, bestehend aus aliphatischen, aromatischen und substituierten aromatischen Sulfonsäuren, Schwefelsäure, Phosphorsäure, sauren Tonen und sauren Molekularsieben.

7. Verfahren nach Anspruch 6, wobei der saure Katalysator eine aliphatische Sulfonsäure ist.

8. Verfahren nach Anspruch 1, wobei die Äquivalente von saurem Katalysator zu Äquivalenten von Benzotriazol 0,2 bis 3 betragen.

9. Stabilisierte Masse, umfassend
(a) ein organisches Material und
(b) als Stabilisator ein Gemisch, erhältlich gemäß Anspruch 1.

10. Stabilisierte Masse nach Anspruch 9, wobei das organische Material (a) ein organisches Polymer ist.

11. Stabilisierte Masse nach Anspruch 10, wobei das organische Polymer ein Polyolefin, ein Styrolpolymer, ein Polyacrylat, ein Polyamid, ein Polyurethan, ein Halogen enthaltendes Vinylpolymer, ein Alkydharz, ein duroplastisches Acrylharz oder ein Epoxidharz ist.

12. Stabilisierte Masse nach Anspruch 11, wobei das organische Material in Form eines photographischen Materials oder Teil eines photographischen Elements vorliegt.

## Revendications (Revendications pour l'(les) Etat(s) contractant(s) suivant(s) : BE, CH, DE, FR, GB, IT, LI, NL)

1. Procédé pour la préparation d'un mélange normalement liquide ou non-cristallin de 2-hydroxyphényl-2H-benzotriazoles, lequel procédé comprend la réaction d'un 2H-benzotriazole de formule (A) ou (B) : 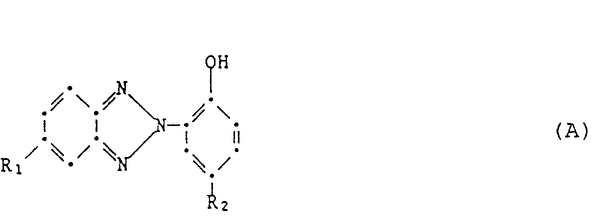 ou 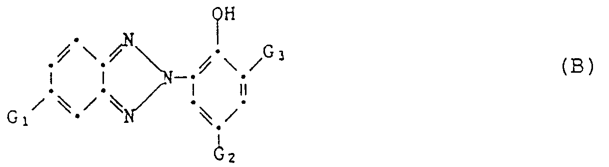 dans laquelle :
R₁ représente les groupes choisis parmis les hydrogène, chlore, alkyle comportant de 1 à 4 atomes de carbone ou alcoxy comportant de 1 à 4 atomes de carbone,
R₂ représente les groupes choisis parmi les alkyle comportant de 2 à 12 atomes de carbone, cycloalkyle comportant de 5 à 8 atomes de car-bone, aralkyle comportant de 7 à 9 atomes de carbone ou chlore,
G₁ ayant la même signification que Ri, et
G₂ et G₃ représentent indepndamment les aralkyle comportant de 7 à 9 atomes de carbone, cycloalkyle comportant de 5 à 8 atomes de carbone, alkyle ramifié comportant de 3 à 12 atomes de carbone, ou l'un des G₂ et G₃ représente les groupes choisis parmi les méthyle, éthyle ou chlore lorsque l'autre des G₂ et G₃ représente les alkyle ramifié, cycloalkyle ou aralkyle ayant la définition donnée ci-dessus,
avec une chaîne alcène linéaire ou ramifiée comportant de 8 à 40 atomes de carbone ou un mélange desdits alcè-nes en présence d'un catalyseur acide à une température de 100 à 200 _{°} C.

2. Procédé selon la revendication 1, dans lequel R₂ ou G₂ et G₃ indépendamment représentent les alkyle ramifié comportant de 3 à 12 atomes de carbone ou aralkyle comportant de 7 à 9 atomes de carbone, ou R₂ représente l'éthyle ou le chlore, ou l'un des G₂ ou G₃ représente les méthyle, éthyle ou chlore et l'autre des G₂ ou G₃ représente un alkyle ramifié comportant de 3 à 12 atomes de carbone.

3. Procédé selon la revendication 1, dans lequel la température est de 140 à 180 ° C.

4. Procédé selon la revendication 1, dans lequel l'alcène comporte de 8 à 16 atomes de carbone.

5. Procédé selon la revendication 1, dans lequel le rapport d'équivalents entre l'alcène et le 2H-benzotriazole est de 1:1 à 6:1.

6. Procédé selon la revendication 1, dans lequel le catalyseur acide est choisi dans le groupe comportant les acides sulfoniques, aliphatiques, aromatiques et aromatiques substitués, l'acide sulfurique, l'acide phosphorique, les argiles acides et les tamis moléculaires acides.

7. Procédé selon la revendication 6, dans lequel le catalyseur acide est un acide sulfonique aliphatique.

8. Procédé selon la revendication 1, dans lequel les équivalents de catalyseur acide par rapport aux équivalents de benzotriazole sont de 0,2 à 3.

9. Mélange normalement liquide ou non-cristallin de benzotriazoles que l'on peut obtenir selon le procédé de la revendication 1.

10. Composition stabilisée comprenant :
(a) une matière organique, et
(b) un mélange tel que défini dans la revendication 9, en tant que stabilisant.

11. Composition stabilisée selon la revendication 10, dans laquelle la matière organique (a) est un polymère organique.

12. Composition stabilisée selon la revendication 11, dans laquelle le polymère organique est les polyoléfine, polymère styrénique, polyacrylate, polyamide, polyuréthanne, polymère vinylique halogéné, résine alkyde, résine acrylique thermodurcissable ou résine époxy.

13. Composition stabilisée selon la revendication 12, dans laquelle la matière organique est présente sous la forme d'un matériau photographique ou fait partie d'un élément photographique.

14. Utilisation d'un mélange tel que défini dans la revendication 9, en tant que stabilisant pour matière organique.

## Revendications (Revendications pour l'(les) Etat(s) contractant(s) suivant(s) : ES)

1. Procédé pour la préparation d'un mélange normalement liquide ou non-cristallin de 2-hydroxyphényl-2H-benzotriazoles lequel procédé comprend la réaction d'un 2H-benzotriazole de formule (A) ou (B) : 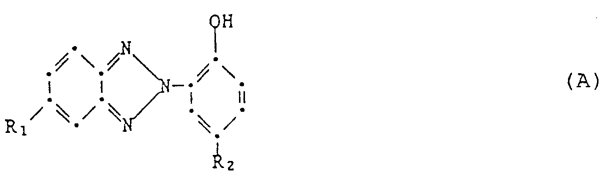 ou 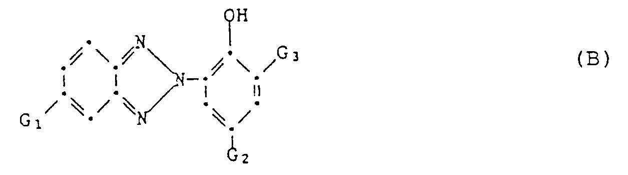 dans laquelle :
R₁ représente les groupes choisis parmi les hydrogène, chlore, alkyle comportant de 1 à 4 atomes de carbone ou alcoxy comportant de 1 à 4 atomes de carbone,
R₂ représente les groupes choisis parmi les alkyle comportant de 2 à 12 atomes de carbone, cycloalkyle comportant de 5 à 8 atomes de carbone, aralkyle comportant de 7 à 9 atomes de carbone ou chlore,
G₁ ayant la même signification que Ri, et
G₂ et G₃ représentent indépendamment les aralkyle comportant de 7 à 9 atomes de carbone, cycloalkyle comportant de 5 à 8 atomes de carbone ou alkyle ramifié comportant de 3 à 12 atomes de carbone, ou l'un des G₂ et G₃ représente les groupes choisis parmi les méthyle, éthyle ou chlore lorsque l'autre des G₂ et G₃ représente les alkyle ramifié, cycloalkyle ou aralkyle ayant la définition donnée ci-dessus,
avec une chaîne alcène linéaire ou ramifiée comportant de 8 à 40 atomes de carbone ou un mélange desdits alcènes en présence d'un catalyseur acide à une température de 100 à 200 _{°} C.

2. Procédé selon la revendication 1, dans lequel R₂ ou G₂ et G₃ indépendamment représentent les alkyle ramifié comportant de 3 à 12 atomes de carbone ou aralkyle comportant de 7 à 9 atomes de carbone, ou R₂ représente l'éthyle ou le chlore, ou l'un des G₂ ou G₃ représente les méthyle, éthyle ou chlore et l'autre des G₂ ou G₃ représente un alkyle ramifié comportant de 3 à 12 atomes de carbone.

3. Procédé selon la revendication 1, dans lequel la température est de 140 à 180 _{°} C.

4. Procédé selon la revendication 1, dans lequel l'alcène comporte de 8 à 16 atomes de carbone.

5. Procédé selon la revendication 1, dans lequel le rapport d'équivalents entre l'alcène et le 2H-benzotriazole est de 1:1 à 6:1.

6. Procédé selon la revendication 1, dans lequel le catalyseur acide est choisi dans le groupe comportant les acides sulfoniques aliphatiques, aromatiques et aromatiques substitués, l'acide sulfurique, l'acide phosphorique, les argiles acides et les tamis moléculaires acides.

7. Procédé selon la revendication 6, dans lequel le catalyseur acide est un acide sulfonique aliphatique.

8. Procédé selon la revendication 1, dans lequel les équivalents de catalyseur acide par rapport aux équivalents de benzotriazole sont de 0,2 à 3.

9. Composition stabilisée qui comprend :
(a) une matière organique, et
(b) un mélange que l'on peut obtenir conformément à la revendication 1, en tant que stabilisant.

10. Composition stabilisée conformément à la revendication 9, dans laquelle la matière organique (a) est un polymère organique.

11. Composition stabilisée conformément à la revendication 10, dans laquelle le polymère organique est une polyoléfine un polymère styrénique, un polyacrylate, un polyamide, un polyuréthanne, un polymère vinylique halogéné, une résine alkyde, une résine acrylique thermodurcissable ou une résine époxy.

12. Composition stabilisée conformément à la revendication 11, dans laquelle la matière organique est sous la forme d'un matériau photographique ou fait partie d'un composant photographique.
